# EUROPEAN PATENT APPLICATION

(11) **EP 4 623 912 A1**
(43) Date of publication of application: **01.10.2025**
(21) Application number: 24166005.9
(22) Date of filing: 25.03.2024
(51) Int. Cl.: A61K 31/426, A61K 31/4985, A61K 31/519, A01N 1/02

(54) **A METHOD FOR DEFATTENING A STEATOTIC LIVER EX VIVO**

(71) Applicant: ETH Zurich, 8092 Zurich (CH)
(72) Inventor: WOLFRUM, Christian, 8104 Weiningen (CH); DING, Lianggong, 8600 Dübendorf (CH); TIBBITT, Mark, 8004 Zürich (CH); HOFFMANN, Waldemar, 79807 Lottstetten (DE); HUWYLER, Florian, 8049 Zürich (CH); BiNZ, Jonas, 5000 Aarau (CH)

(57) **Abstract**

The present invention relates to a method for defattening a steatotic liver *ex vivo*, the method comprising perfusing a steatotic liver graft in a perfusion step using a perfusate comprising a PI4KB inhibitor as specified or a pharmaceutically acceptable salt or solvate thereof.

## Description

### Field

The present invention relates to a method for defattening a steatotic liver ex *vivo* by perfusion using a PI4KB inhibitor.

### Background

The catabolism of the main nutrients glucose, amino acids and fatty acids (FAs) is a central function of almost all cell types to maintain energy requirements for their various cellular activities. Among these nutrients, FAs have the highest energy density and thus constitute an important long-term cellular energy substrate. Due to the toxicity of FAs at high concentrations, most of them are esterified into triglycerides (TGs) and stored in lipid droplets (LDs). To circumvent nutritional shortage during starvation, the TG stores are mobilized to liberate FAs to meet cellular energy demands via lipolysis or autophagic degradation of LDs (i.e., lipophagy). Lipolysis is executed sequentially by adipose triglyceride lipase (ATGL), hormone-sensitive lipase (HSL) and monoacylglycerol lipase (MGL) to hydrolyze TG into FAs and glycerol, while the process of lipophagy is controlled by lysosomal acid lipase (LAL). Although LDs can be generated in almost any cell types to store excess intracellular FAs, adipose tissue is the organ with the highest level of LD formation and storage. In the presence of an adequate glucose supply, insulin signaling increases lipogenesis while repressing lipolysis to promote lipid storage in the adipose tissue. Conversely, hormonal (epinephrine) or sympathetic-neuronal (norepinephrine) stimulation activates adipocytic lipolysis to supply other organs with FAs during the fasting state.

Among the three lipases involved in lipolysis, HSL is activated by phosphorylation through catecholamine signaling activated protein kinase A (PKA). Conversely, insulin suppresses this process via phosphodiesterase 3B (PDE3B) mediated cAMP degradation. Nevertheless, loss of HSL leads to mild phenotypes in both humans and mice. Thus, liver-specific depletion of HSL does not impact hepatic TG levels and in adipose tissue loss of HSL leads to an age-dependent hepatic steatosis as a result of progressive lipodystrophy. In contrast, ATGL plays a broader role as exemplified by substantial LD accumulation in various tissues from humans or mice with *Atgl* loss of function. Moreover, liver-specific *Atgl* knockout in mice promotes hepatic LD accumulation, whereas an adipose tissue-specific *Atgl* knockout displays substantially reduced neutral lipid levels in the liver, underscoring the role of ATGL in providing both intracellular and extracellular FAs. ATGL function is modulated at multiple levels to meet the complex physiological demands of energy homeostasis. Transcriptionally, insulin signaling inhibits *ATGL* mRNA synthesis through suppression of FOXO1. At the post-translational level, catecholamine and glucagon signaling promote ATGL enzymatic activity through PKA- and CAMKII-mediated phosphorylation of ATGL at Ser406 in adipocytes and hepatocytes. In addition, ATGL abundance can be regulated by proteasome-mediated degradation after polyubiquitination via different E3 ligases, such as COP1 and PEX2.

The Golgi apparatus is defined by tethered stacks of cisternae with myriad intracellular functions, including protein modification and folding, vesicle formation, cytoskeleton organization and lipid metabolism. To execute these processes, the Golgi apparatus incorporates integral proteins spanning the membrane or recruits cytosolic proteins via interaction with Golgi-enriched lipids, such as Ptdlns4P. Owing to its positively charged property, Ptdlns4P can interact with proteins containing negatively charged regions, such as NLRP3 and STUB1, thereby enabling Golgi Ptdlns4P to regulate inflammation and protein degradation, respectively. The Golgi Ptdlns4P pool is generated by the lipid kinases PI4KB, PI4K2A and PI4K2B to generate Ptdlns4P from Ptdlns. Conversely, the lipid phosphatase SACM1L dephosphorylates Ptdlns4P. SACM1L activity is modulated via altered distribution in the ER and the Golgi, and enrichment of SACM1L in the latter compartment is promoted by glucose or serum deprivation, leading to a reduction of Ptdlns4P levels in the Golgi apparatus, which might affect pleiotropic intracellular processes via Ptdlns4P interactors.

To date, hormone signaling is still the best documented mechanism to coordinate glucose availability and systemic lipolysis. In the context of the present invention, general cell-intrinsic mechanisms that balance the supply of glucose and FAs levels by regulation of lipolysis via intracellular glucose sensing were investigated. Intriguingly, such a novel regulatory mechanism involving the central role of Ptdlns4P in the assembly of the E3 ligase complex CUL7^{FBXW8} in the Golgi apparatus, which in turn polyubiquitylates ATGL for proteasome-targeted degradation, was identified. Notably, depletion of intracellular glucose levels is sensed by this pathway, leading to reduced assembly of the E3 ligase complex and increased ATGL-driven lipolysis. In this manner, the pools of glucose and FAs are intertwined.

Based on the above-mentioned state of the art, the objective of the present invention is to provide means and methods for defattening a steatotic liver ex *vivo.* This objective is attained by the subject-matter of the independent claims of the present specification, with further advantageous embodiments described in the dependent claims, examples, figures and general description of this specification.

### Summary of the Invention

A first aspect of the invention relates to method for defattening a steatotic liver ex *vivo,* the method comprising perfusing a steatotic liver graft in a perfusion step using a perfusate comprising a compound selected from a PI4KB inhibitor of formula 1, 2, and 3, or a pharmaceutically acceptable salt or solvate thereof, 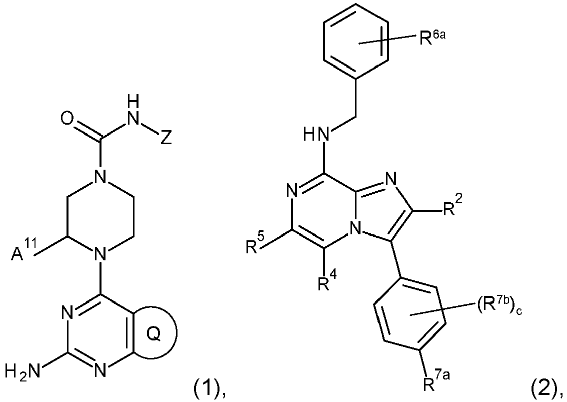 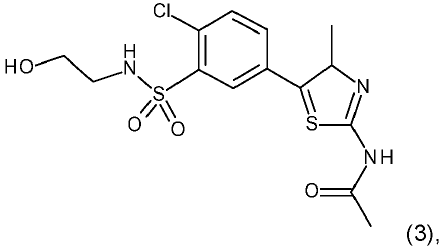 wherein
- Z is (methoxy)(methyl)phenyl, (methyl)(trifluoromethoxy)phenyl, (methoxy)(methyl)pyridinyl, (ethyl)(methoxy)pyridinyl, (ethoxy)(methyl)pyridinyl or dimethoxypyridinyl,
- A¹¹ is H, methyl, ethyl, hydroxymethyl or hydroxyethyl,
- Q is a heterocycle of formula 4, 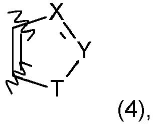 wherein
   X is N or C(H),
   Y is N or C(H),
   T is S or N(CH₃)
- R², R⁴ and R⁵ are independently selected from H, methyl and ethyl,
- R^{6a} is SO₂R⁸, wherein R^{6a} is in para or meta position,
- R^{7a} is selected from OH, OCH₃, halogen, NH₂, CH₂-OH and NH-CO-R⁸,
- each R^{7b} is independently selected from OH, halogen, CN, NO₂, C₁-C₆ alkyl, C₁-C₆ alkoxy, OCF₃, OCH₂CF₃, OCH₂Ph, OCH₂-cyclopropyl, OCH₂CH₂OH, OCH₂CH₂NMe₂, CF₃, OCF₃, C₁-C₆ alkyl-OH, NHSO₂R⁹, NHCOR⁸ and NR⁹R¹⁰,
- c is 0 or 1
   wherein
   R⁸ is C₁-C₆ alkyl or NR⁹R¹⁰
   R⁹ and R¹⁰ are independently selected from H and C₁-₆ alkyl.

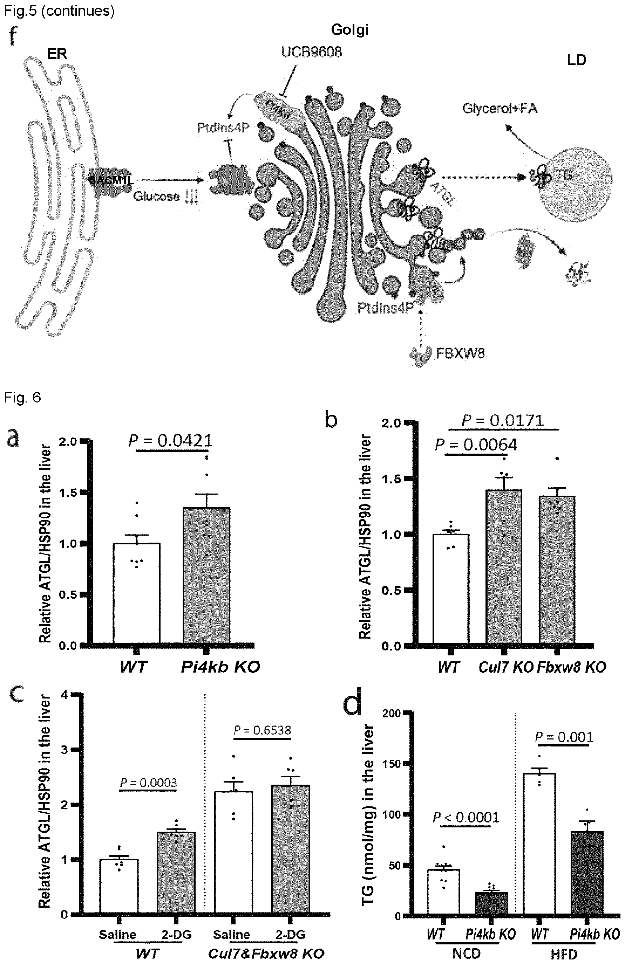

The present invention makes use of a cell-intrinsic mechanism, which involves Golgi Ptdlns4P-mediated regulation of adipose triglyceride lipase (ATGL) driven lipolysis via intracellular glucose sensing (see Fig. 5f). Mechanistically, depletion of intracellular glucose results in lower Golgi Ptdlns4P levels, and thus reduced assembly of the E3 ligase complex CUL7^{FBXW8} in the Golgi apparatus as the assembly of the E3 ligase complex requires the presence of Ptdlns4P. Decreased levels of the E3 ligase complex lead to reduced polyubiquitylation of ATGL in the Golgi and thus reduced proteasome-mediated degradation. As a consequence, the ATGL level increases and ATGL-driven lipolysis is enhanced. This cell-intrinsic mechanism regulates both the pool of intracellular fatty acids (FAs) and their extracellular release to meet physiological demands, including fasting and glucose deprivation.

Enhancement of ATGL-driven lipolysis cannot only be observed after depletion of intracellular glucose levels but also be achieved by inhibiting the lipid kinase PI4KB that generates Ptdlns4P from Ptdlns. The compounds as claimed are PI4KB inhibitors that are useful in the pharmacological manipulation of the Golgi PtdIns4P-CUL7^{FBXW8}-ATGL axis as shown in mouse models of simple hepatic steatosis and NASH. Administration of a PI4KB inhibitor as claimed resulted in therapeutic amelioration, i.e. defattening of steatotic mouse livers. Similarly, defattening of a human steatotic liver graft was achieved by ex *vivo* perfusion using a PI4KB inhibitor as claimed.

### Terms and definitions

### General

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

### Specific terms

The term *fatty liver disease* relates to a condition where excess fat builds up in the liver. Synonymous terms are *hepatic steatosis* and *steatotic liver disease (SLD).*

In the context of the present specification, the term fatty liver disease encompasses hepatic steatosis conditions without inflammation (also referred to as "simple hepatic steatosis") as well as hepatic steatosis conditions with inflammation (steatohepatitis).

Hepatic steatosis is caused by an impairment of the fatty acid metabolism which leads to the accumulation of triglyceride fat in the cytoplasm. The diagnosis is made when fat in the liver exceeds 5-10% by weight.

At the beginning, the hepatocytes present liposomes around the nucleus without displacement of the centrally located nucleus (microvesicular fatty change). In late stages, the size of the liposomes increases and the nucleus dislocates to the periphery of the cell, giving a characteristic signet ring appearance (macrovesicular fatty change). Macrovesicular steatosis is the most common form and is typically associated with alcohol, diabetes, obesity, and corticosteroids. Acute fatty liver of pregnancy and Reye's syndrome are examples of hepatic steatosis caused by microvesicular fatty change.

Severe fatty liver is sometimes accompanied by inflammation, a situation referred to as steatohepatitis. Progression to alcoholic steatohepatitis (ASH) or non-alcoholic steatohepatitis (NASH) depends on the persistence or severity of the underlying cause. Pathological lesions in both conditions are similar. Steatosis (retention of lipid) and onset of steatohepatitis may represent successive stages in fatty liver disease progression.

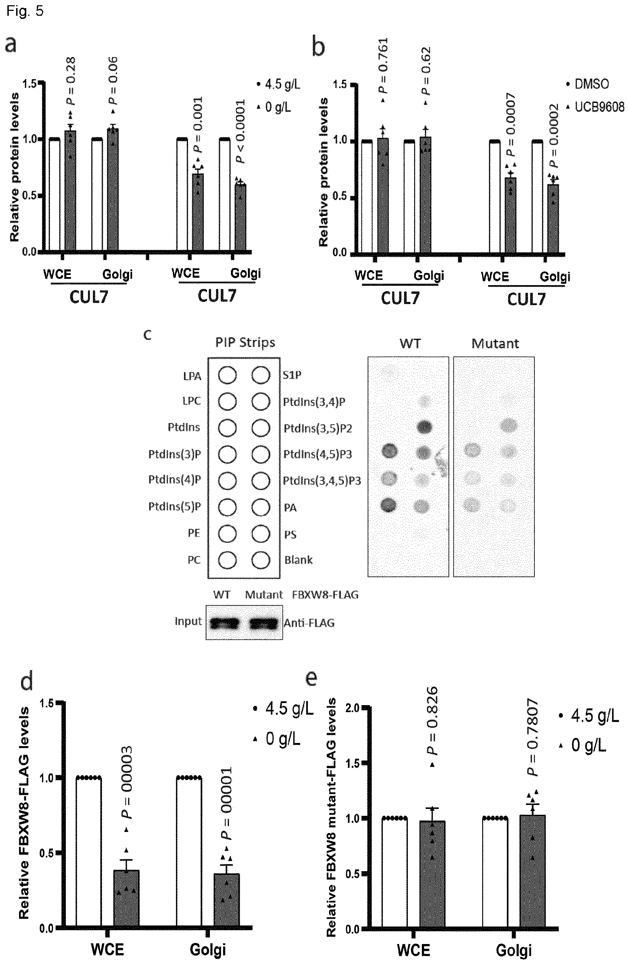

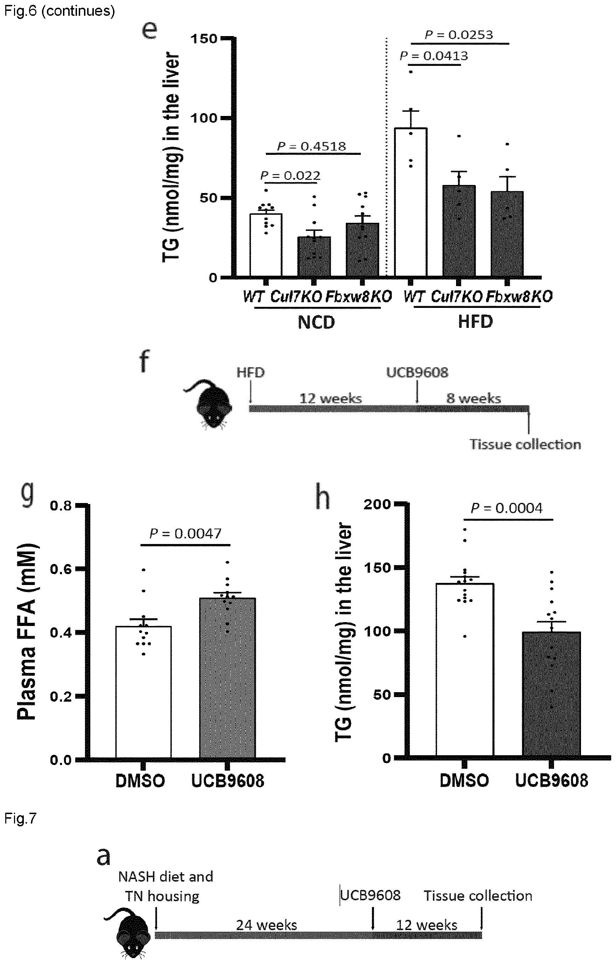

Based on different aetiologies, fatty liver disease may be classified into the following subtypes (Renella et al. 2023, Hepatology 78(6):1966-1986, particularly Fig. 5 and Fig. 6):
- metabolic dysfunction-associated steatotic liver disease (MASLD),
- metabolic and alcohol associated liver disease (metALD),
- alcohol-associated liver disease (ALD)
- cryptogenic steatotic liver disease,
- steatotic liver disease with specific aetiology.

In the context of the present specification, terms such as MASLD, metALD or ALD refer to hepatic steatosis conditions without inflammation.

When accompanied by inflammation, MASLD may progress to metabolic dysfunction-associated steatohepatitis (MASH). Similarly, metALD and ALD may progress to metabolic and alcohol associated steatohepatitis and alcoholic steatohepatitis (ASH), respectively.

Cryptogenic steatotic liver disease relates to fatty liver disease with no identifiable cause. It may progress to cryptogenic steatohepatitis.

SLD with specific aetiology includes for instance drug-induced or monogenic fatty liver disease. SLD with specific aetiology may progress to steatohepatitis with specific aetiology. The terms *metabolic dysfunction-associated steatotic liver disease* (MASLD) and *non-alcoholic fatty liver disease* (NAFLD) are used interchangeably.

The terms *metabolic dysfunction-associated steatohepatitis* (MASH) and *non-alcoholic steatohepatitis* (NASH) are used interchangeably.

The term *PI4KB* in the context of the present specification relates to phosphatidylinositol 4-kinase beta, particularly to the proteins referred to by UniProt identifiers Q9UBF8 (human PI4KB) and Q8BKC8 (mouse). The phosphatidylinositol 4-kinase beta catalyses phosphorylation of phosphatidylinositol at the D-4 position yielding phosphatidylinositol-4-phosphate.

The term *Ptdlns4P* in the context of the present specification relates to phosphatidylinositol-4-phosphate.

The term *Ptdlns* in the context of the present specification relates to phosphatidylinositol.

The term ATGL in the context of the present invention relates to adipose triglyceride lipase, particularly to the proteins referred to by UniProt identifiers Q96AD5 (human) and Q8BJ56 (mouse).

The term *IGL-1* solution relates to solution having the following composition:
Lactobionic acid : 35,8 g/L; Adenosine : 1,336 g/L; Allopurinol : 0,136 g/L; Glutathione : 0,922 g/L; Polyethylene glycol (PEG 35000) : 1 g/L; Potassium dihydrogen phosphate : 3,402 g/L; Raffinose pentahydrate : 17,84 g/L; Magnesium sulfate heptahydrate : 1,232 g/L; Sodium Hydroxide : qs; pH : 7.4; Water for injection : qs 1 liter.

IGL-1 is a clear, sterile, non-pyrogenic solution intended for hypothermic flushing and storage of organs (kidneys, liver, pancreas). IGL-1 solution has an osmolality of 290 mOsm/kg, a total sodium concentration of 120 mmol/L, a total potassium concentration of 25 mmol/L and a pH of 7.4 at room temperature.

The term *Belzer MPS* relates to a solution having the following composition:

| | | |
|---|---|---|
| Calcium chloride (dihydrate) | 0.068 g | 0,5 mmol/L |
| HEPES (free acid) | 2.38 g | 10 mmol/L |
| Potassium phosphate (monobasic) | 3.4 g | 25 mmol/L |
| Mannitol (USP) | 5.4 g | 30 mmol/L |
| Glucose (anhydrous), beta D (+) | 1.80 g | 10 mmol/L |
| Sodium gluconate | 17.45 g | 80 mmol/L |
| Magnesium gluconate (anhydrous | 1.13 g | 5 mmol/L |
| Ribose, D (-) | 0.75 g | 5 mmol/L |
| Pentafraction (HES) | 50.0 g | 50 g/L |
| Glutathione (reduced form) | 0.92 g | 3 mmol/L |
| Adenine (free base) | 0.68 g | 5 mmol/L |
| Sodium Hydroxide | | qs pH: 7.4 |
| Water for injection | | qs 1000 mL |

Belzer MPS is a clear to slightly yellow, sterile, non-pyrogenic, non-toxic solution for the in-vitro flushing and temporary continuous perfusion preservation of the explanted organs. This solution has an approximate calculated osmolarity of 300 mOsm/kg, potassium concentration of 25 mmol/L, sodium 100 mmol/L and a pH of approximately 7.4 at room temperature.

The term *Belzer UW solution* (University of Wisconsin Solution) relates to a solution having the following composition:

| | | |
|---|---|---|
| Pentafraction (HES) | 50 g | 50 g/L |
| Lactobionic Acid (as Lactone) | 35.83 g | 105 mmol/L |
| Potassium Phosphate monobasic | 3.40 g | 25 mmol/L |
| Magnesium Sulfate heptahydrate | 1.23 g | 5 mmol/L |
| Raffinose pentahydrate | 17.83 g | 30 mmol/L |
| Adenosine | 1.34 g | 5 mmol/L |
| Allopurinol | 0.136 g | 1 mmol/L |
| Total Glutathione | 0.922 g | 3 mmol/L |
| Potassium Hydroxide | 5.61 g | 100 mmol/L |
| Sodium Hydroxide /Hydrochloric Acid | | qs pH: 7.4 |
| Water for Injection | | qs 1000 mL |

The term *Williams E* relates to a cell culture medium as described in Williams, G.M., and Gunn, J.M., Long-Term Cell Culture of Adult Rat Liver Epithelial Cells. Exp. Cell Research, 89, 139-142 (1974) and Williams, G.M. et al., Isolation and Long-Term Cell Culture of Epithelial-Like Cells From Rat Liver. Exp. Cell Research, 69, 106-112 (1971).

The term *DMEM* relates to a cell culture medium as described in Dulbecco, R., and Freeman, G., Plaque Production by the Polyoma Virus. Virology, 8, 396-397 (1959); Smith, J.D., Freeman, G., Vogt, M., and Dulbecco, R., The Nucleic Acid of Polyoma. Virus, 12, 185-196 (1960); Morton, H.J., A Survey of Commercially Available Tissue Culture Media. In Vitro, 6, 89 (1970); Rutzky, L.P., and Pumper, R.W., Supplement to a Survey of Commercially Available Tissue Culture Media (1970). In Vitro, 9, 468 (1974).

### General Molecular Biology: Nucleic Acid Sequences, Expression

The term *gene* refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The term *recombinant* in the context of the present specification relates to a nucleic acid, which is the product of one or several steps of cloning, restriction and/or ligation and which is different from the naturally occurring nucleic acid. A recombinant virus particle comprises a recombinant nucleic acid.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

### Inhibition

The term *inhibitor* in the context of the present specification relates to any pharmaceutically acceptable agent or compound that may be used to interact with and specifically interfere with the biological activity of its designated target (phosphatidylinositol 4-kinase, specifically PI4KB in the case of the present specification). Inhibitors include small molecule drugs that fulfil the criteria summarized as Lipinski's Rules of five (the drug fulfils at least three of the following rules: number of H-bond donors is ≤ 5; number of H-bond acceptors is ≤ 10; molecular mass is <500Da; octanol/water partition coefficient ≤ 5). Specific examples of such inhibitors are mentioned herein.

The term *PI4KBK inhibitor* in the context of the present invention relates to a compound that may be used to interfere with the biological activity of phosphatidylinositol 4-kinase beta (PI4KB), i.e. the phosphorylation of phosphatidylinositol to generate phosphatidylinositol-4-phosphate. The term *PI4KBK inhibitor* includes the compounds of formula 1, 2 and 3 as claimed. Particular examples of PI4KB inhibitors are UCB9608, BF738735 and PIK93:

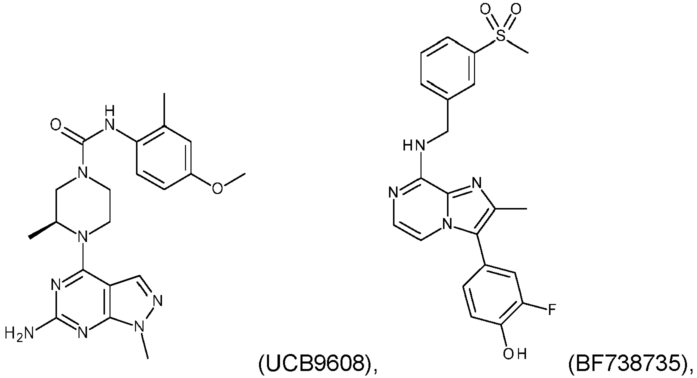

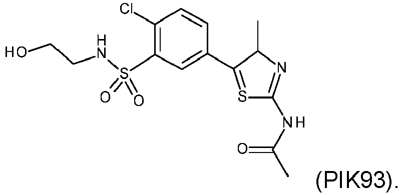

### Treatment

As used herein, the term *treating* or *treatment* of any disease or disorder (e.g. NAFLD or NASH) refers in one embodiment to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow. In the context of the present invention, treatment of fatty liver disease relates in particular to the reduction of hepatic triglyceride levels and/or the prevention of progression of NASH from simple hepatic steatosis.

### Organic Chemistry

The formulae of the present specification follow the convention of organic chemistry to not show hydrogen atoms on carbon scaffolds. Carbon is tetravalent and bonds not shown are assumed to be hydrogen unless shown otherwise. Hydrogen can be exchanged for deuterium without changing the bulk chemical properties of the molecule; however, in the case of dye or drug molecules, the exchange of hydrogen for deuterium may lead to changes in the spectral properties or receptor interactions of the molecule. Unless explicitly stated otherwise herein, the disclosure of a formula showing, explicitly or implicitly by the convention restated in the first sentence of this paragraph, encompasses molecules in which one or several of the hydrogen atoms are exchanged for deuterium.

The term *(methoxy)(methyl)phenyl* relates to a phenyl moiety substituted by a methoxy (-O-CH₃) and a methyl (-CH₃) substituent. Similarly, *(methyl)(trifluoromethoxy)phenyl* relates to a phenyl moiety substituted by a methyl substituent and a trifluoromethoxy (-O-CF₃) substituent.

The term *(methoxy)(methyl)pyridinyl* relates to a pyridinyl moiety substituted by a methoxy (-O-CH₃) substituent and a methyl (-CH₃) substituent. Similarly, *(ethyl)(methoxy)pyridinyl, (ethoxy)(methyl)pyridinyl*and *dimethoxypyridinyl* relate to pyridinyl moieties substituted by an ethyl (-CH₂-CH₃), methoxy (-O-CH₃), ethoxy (-O-CH₂-CH₃), methyl (-CH₃) or two methoxy (-O-CH₃) moieties, respectively.

The terms *hydroxymethyl* and *hydroxyethyl* relate to -CH₂-OH and -CH₂-CH₂-OH, respectively.

The term *halogen* refers to a member of the group fluorine, chlorine, bromine and iodine. In particular embodiments, the term refers to a member of the group chlorine, bromine and iodine. In other particular embodiments, the term refers to a member of the group chlorine and bromine.

The term *alkyl* in the context of the present specification relates to a saturated linear, branched or (partially or completely) cyclic hydrocarbon. Particularly, the term *alkyl* relates to a saturated linear hydrocarbon.

The term C₁-C₄ *alkyl* in the context of the present specification relates to a saturated linear or branched hydrocarbon having 1, 2, 3 or 4 carbon atoms. Non-limiting examples for a C₁-C₄ alkyl are methyl, ethyl, propyl, prop-2-enyl, n-butyl, 2-methylpropyl, tert-butyl, cyclo-butyl, cyclo-propyl, methyl-cyclo-propyl. In certain embodiments, a C₁-C₄ alkyl is a methyl, ethyl, propyl or butyl moiety.

A C₁-C₆ *alkyl* in the context of the present specification relates to a saturated linear or branched hydrocarbon having 1, 2, 3, 4, 5 or 6 carbon atoms. Non-limiting examples for a C₁-C₆ alkyl include the examples given for C₁-C₄ alkyl above, and additionally n-pentyl, 2-methylbutyl, 3-methylbutyl, 1,1-dimethylpropyl, 1,2-dimethylpropyl, 1,2-dimethylpropyl, cyclo-pentyl, cyclohexyl, methyl-cyclo-pentyl. In certain embodiments, a C₅ alkyl is a pentyl or cyclopentyl moiety and a C₆ alkyl is a hexyl or cyclohexyl moiety.

Where used in the context of chemical formulae, the following abbreviations may be used: Me is methyl CH₃, *Et* is ethyl -CH₂CH₃, *Prop* is propyl -(CH₂)₂CH₃ (n-propyl, n-pr) or -CH(CH₃)₂ (iso-propyl, i-pr), *but* is butyl -C₄H₉, -(CH₂)₃CH₃, -CHCH₃CH₂CH₃, -CH₂CH(CH₃)₂ or -C(CH₃)₃.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable* carrier includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624). The invention also encompasses nanoparticles, liposomes, or cellular carriers within the meaning of *pharmaceutically acceptable carrier.*

### Detailed Description of the Invention

A first aspect of the invention relates to method for defattening a steatotic liver ex *vivo,* the method comprising
perfusing a steatotic liver graft in a perfusion step using a perfusate comprising a compound selected from a PI4KB inhibitor of formula 1, 2, and 3, or a pharmaceutically acceptable salt or solvate thereof, 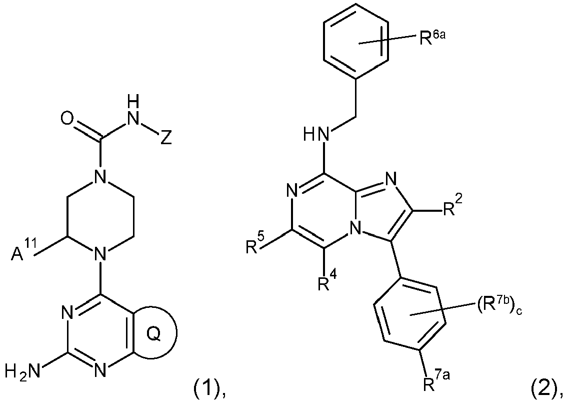 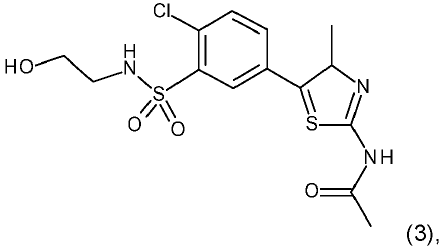 wherein
- Z is (methoxy)(methyl)phenyl, (methyl)(trifluoromethoxy)phenyl, (methoxy)(methyl)pyridinyl, (ethyl)(methoxy)pyridinyl, (ethoxy)(methyl)pyridinyl or dimethoxypyridinyl,
- A¹¹ is H, methyl, ethyl, hydroxymethyl or hydroxyethyl,
- Q is a heterocycle of formula 4, 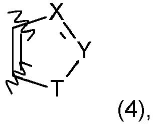 wherein
   X is N or C(H),
   Y is N or C(H),
   T is S or N(CH₃)
- R², R⁴ and R⁵ are independently selected from H, methyl and ethyl,
- R^{6a} is SO₂R⁸, wherein R^{6a} is in para or meta position,
- R^{7a} is selected from OH, OCH₃, halogen, NH₂, CH₂-OH and NH-CO-R⁸,
- each R^{7b} is independently selected from OH, halogen, CN, NO₂, C₁-C₆ alkyl, C₁-C₆ alkoxy, OCF₃, OCH₂CF₃, OCH₂Ph, OCH₂-cyclopropyl, OCH₂CH₂OH, OCH₂CH₂Nme₂, CF₃, OCF₃, C₁-C₆ alkyl-OH, NHSO₂R⁹, NHCOR⁸ and NR⁹R¹⁰,
- c is 0 or 1
   wherein
   R⁸ is C₁-C₆ alkyl or NR⁹R¹⁰
   R⁹ and R¹⁰ are independently selected from H and C₁-₆ alkyl.

Another embodiment of the invention relates to method for defattening a steatotic liver ex *vivo,* the method comprising perfusing a steatotic liver graft in a perfusion step using a perfusate comprising a PI4KB inhibitor or a pharmaceutically acceptable salt or solvate thereof.

The term (methoxy)(methyl)phenyl relates to a phenyl moiety substituted by a methoxy (-O-CH₃) and a methyl (-CH₃) substituent. Similarly, (methyl)(trifluoromethoxy)phenyl relates to a phenyl moiety substituted by a methyl substituent and a trifluoromethoxy (-O-CF₃) substituent.

The term (methoxy)(methyl)pyridinyl relates to a pyridinyl moiety substituted by a methoxy (-O-CH₃) substituent and a methyl (-CH₃) substituent. Similarly, (ethyl)(methoxy)pyridinyl, (ethoxy)(methyl)pyridinyl and dimethoxypyridinyl relate to pyridinyl moieties substituted by an ethyl (-CH₂-CH₃), methoxy (-O-CH₃), ethoxy (-O-CH₂-CH₃), methyl (-CH₃) or two methoxy (-O-CH₃) moieties, respectively.

In certain embodiments, the compound is selected from a PI4KB inhibitor of formula 1, 2, and 3, or a pharmaceutically acceptable salt or solvate thereof.

In certain embodiments, the compound is selected from a PI4KB inhibitor of formula 1, 2, and 3.

In certain embodiments, the compound is a PI4KB inhibitor of formula 1.

In certain embodiments, the steatotic liver graft is a human steatotic liver graft.

In certain embodiments, the concentration of the PI4KB inhibitor in the perfusate is 0.1 to 100 µM.

In certain embodiments, the concentration of the PI4KB inhibitor in the perfusate is 1 to 100 µM.

In certain embodiments the concentration of the PI4KB inhibitor in the perfusate is 0.1 to 40 µM.

In certain embodiments, the concentration of the PI4KB inhibitor in the perfusate is 1 to 40 µM.

In certain embodiments, the concentration of the PI4KB inhibitor in the perfusate is 5 to 40 µM.

In certain embodiments, the concentration of the PI4KB inhibitor in the perfusate is 10 to 40 µM.

In certain embodiments, the concentration of the PI4KB inhibitor in the perfusate is 0.1 to 20 µM.

In certain embodiments, the concentration of the PI4KB inhibitor in the perfusate is 1 to 20 µM.

In certain embodiments, the concentration of the PI4KB inhibitor in the perfusate is 10 to 20 µM.

To get into a therapeutic window, the initial concentration of the PI4KB inhibitor may be higher than the targeted concentration during subsequent perfusion. Typically, the perfusate undergoes hemodialysis during the perfusion. To account for losses due to hemodialysis where part of the PI4KB inhibitor (and other added compounds) are washed out over time, additional boli are added every day to stay in the therapeutic window. Depending on how the PIK4B inhibitor (or another added compound) is dissolved, it may be also administered with continuous infusion via syringe pumps.

In certain embodiments, the initial concentration of the PI4KB inhibitor in the perfusate is 0.1 to 100 µM.

In certain embodiments, the initial concentration of the PI4KB inhibitor in the perfusate is 1 to 100 µM.

In certain embodiments, the initial concentration of the PI4KB inhibitor in the perfusate is 10 to 100 µM.

In certain embodiments, the initial concentration of the PI4KB inhibitor in the perfusate is 0.1 to 40 µM.

In certain embodiments, the initial concentration of the PI4KB inhibitor in the perfusate is 1 to 40 µM.

In certain embodiments, the initial concentration of the PI4KB inhibitor in the perfusate is 10 to 40 µM.

In certain embodiments, the initial concentration of the PI4KB inhibitor in the perfusate is 20 µM.

In certain embodiments, the concentration of the PI4KB inhibitor is controlled and adjusted during the perfusion step to a concentration in the perfusate of 0.1 to 20 µM.

In certain embodiments, the concentration of the PI4KB inhibitor is controlled and adjusted during the perfusion step to a concentration in the perfusate of 1 to 20 µM.

In certain embodiments, the concentration of the PI4KB inhibitor is controlled and adjusted during the perfusion step to a concentration in the perfusate of 5 to 20 µM.

In certain embodiments, the concentration of the PI4KB inhibitor is controlled and adjusted during the perfusion step to a concentration in the perfusate of 10 µM.

The PI4KB inhibitor, particularly UCB9608, may be added in form of a bolus. UCB9608 dissolved in DMSO precipitated when mixed with water and not circulated. Therefore, the PI4KB inhibitor, particularly UCB9608, may be added in form of boli instead of continuous administration with a syringe pump to avoid precipitation within the syringe.

Dose adjustments are dependent on the dialysis flow rate and dialysis filter. Clearance of compounds can be best measured with LC-MS for a specific dialysis filter and flow rate. The dose can then be adjusted accordingly by administering boli or a continuous infusion.

Typically, 1.5 to 3.5 L perfusate are used for perfusion. To target a concentration of 10 µM, a bolus of 15 µmol PI4KB inhibitor, particularly UCB9608, may be added to 1.5 L perfusate. For convenient handling, the PI4KB inhibitor may be dissolved in a suitable solvent, e.g. DMSO, before it is added to the perfusate.

If the PI4KB inhibitor such as UCB9608 is water insoluble, water solubility may be achieved by encapsulation of PI4KB inhibitors or complex formation with albumin. Administration via encapsulation or administration as a bound complex with albumin provides the benefit that it prevents wash-out via dialysis bypasses in perfusion loops, decreases the risk of precipitation in perfusate reservoirs and potentially improves cellular uptake of the inhibitors.

In certain embodiments, the concentration of the PI4KB inhibitor is adjusted by
- adding a bolus of the PI4KB inhibitor, wherein the PI4KB inhibitor is dissolve in an organic solvent, particularly DMSO, or
- continuous administration, wherein the PI4KB inhibitor is bound to albumin, or the PI4KB inhibitor is encapsulated in nanoemulsion, or the PI4KB inhibitor is part of a nanoparticle.

In certain embodiments, the concentration of the PI4KB inhibitor is adjusted by adding a daily bolus.

In certain embodiments, the concentration of the PI4KB inhibitor is adjusted by adding a daily bolus of 10 to 100 µmol per L perfusate.

In certain embodiments, the concentration of the PI4KB inhibitor is adjusted by adding a daily bolus of 10 to 40 µmol per L perfusate.

In certain embodiments, the concentration of the PI4KB inhibitor is adjusted by adding a daily bolus of 10 µmol per L perfusate.

In certain embodiments, the PI4KB inhibitor is dissolved in a suitable solvent.

In certain embodiments, the PI4KB inhibitor is dissolved in an organic solvent.

In certain embodiments, the PI4KB inhibitor is dissolved in DMSO (dimethyl sulfoxide).

In certain embodiments, the daily bolus of the PI4KB inhibitor is added dropwise.

In certain embodiments, the daily bolus of the PI4KB inhibitor is added dropwise within one minute.

For liver transplantation, the perfusion is performed until a non-steatotic liver is obtained. Liver viability and function is determined by a variety of perfusate surrogate markers and perfusion parameters that are known to a person of skill in the art. Related to defatting of organs, punch-biopsies are the most reliable way to see the therapeutic/defattening effect. Non-limiting examples to visualize the lipid content in the biopsies are measurement of triglycerides of snap frozen tissue, or histological assessment with simple H&E staining or lipid specific staining such as Nile Red or oil red O staining. This allows for reassessment of micro- and macrovesicular steatosis of tissue. Transplantation centers have their own thresholds for micro- and macrovesicular steatosis for selecting organs for transplantation. Apart from invasive biopsy related assessment, the whole organ weight can be taken as an indicator of defatting. Further, non-esterified fatty acids (NEFAs) can be measured in waste dialysate.

In certain embodiments, the perfusion step is performed until a non-steatotic liver is obtained.

In certain embodiments, the perfusion step is performed for 2 to 14 days.

In certain embodiments, the perfusion step is performed for 6 to 10 days.

In certain embodiments, the perfusion step is performed for 8 days.

Most perfusion protocols use leukocyte depleted blood as perfusion medium. Leukocyte depleted blood may be obtained by separating leukocytes from whole blood. Suitable methods are known to a person of skill in the art. For instance, leukocytes can be separated from whole blood on the basis of size, dielectric properties, by affinity separation, freeze-thawing, centrifugation or filtration. The blood may be obtained from the organ donor and used as whole blood or leukocyte depleted blood.

Leukocyte-free blood may be obtained by mixing red blood cells, fresh frozen plasma and thrombocytes. Leukocytes are not present.

Alternatively, oxygen-carrying blood substitutes may be used as perfusion medium. An oxygen-carrying blood substitute may be obtained by mixing red blood cells with an organ preservation solution or cell medium. Non-limiting examples for organ preservation solutions are MPS Belzer, IGL-1, UW. Non-limiting examples for suitable cell media are Williams E or DMEM.

Instead of red blood cells, synthetic oxygen carriers such as the ones from HbO2 therapeutics (glutaraldehyde-polymerized bovine haemoglobin) and Hemarina (extracellular haemoglobin from *Arenicola marina*) may be used.

Generally, the perfusion medium should match the blood group of the donor. If not possible, another blood group that is compatible may be used. For example, a liver of a doner with blood group A+ is perfused with a perfusion medium comprising blood cells of the blood group A+.

In certain embodiments, the perfusate comprises a perfusion medium, wherein the perfusion medium is whole blood, leukocyte depleted blood, leukocyte-free blood or an oxygen-carrying blood substitute.

In certain embodiments, the perfusate comprises a perfusion medium, wherein the perfusion medium is leukocyte depleted blood or leukocyte-free blood.

In certain embodiments, the perfusate comprises a perfusion medium, wherein the perfusion medium is leukocyte-free blood. Leukocyte-free blood is particularly obtained by mixing 6 units of red blood cell concentrate (e.g. 200 to 250 mL / Unit), 5 units of fresh frozen blood cells (e.g. 200 mL/Unit), one unit of thrombocytes (e.g. 175 to 400 mL/Unit) and 200 mL of 20% human albumin (CSL Behring).

The hematocrit in the final perfusate after all blood conserves have been mixed is about 30%. In certain embodiments, the hematocrit level of the perfusate is in physiological range, particularly between 25 and 35%, more particularly 30%.

In certain embodiments, the whole blood is donor autologous whole blood.

In certain embodiments, the leukocyte depleted blood is donor autologous leukocyte depleted blood.

In certain embodiments, the oxygen-carrying blood substitute
- is an organ preservation solution or cell medium comprising red blood cells, and/or
- is a solution comprising extracellular haemoglobin or a haemoglobin-based oxygen carrier (HBOC).

In certain embodiments, the extracellular haemoglobin is extracellular haemoglobin from *Arenicola marina.*

In certain embodiments, the HBOC is glutaraldehyde-polymerized haemoglobin.

As described above, the perfusate undergoes hemodialysis during the perfusion to allow for controlling and/or correcting parameters such as the concentration of the PI4K inhibitor and other additives, the pH, hematocrit level or removing waste products.

In certain embodiments, the perfusate undergoes dialysis during the perfusion step.

In certain embodiments, the perfusate undergoes dialysis during the perfusion step for controlling and/or correcting the concentration of the PI4K inhibitor.

In certain embodiments, the perfusate undergoes dialysis during the perfusion step for
- controlling and/or correcting the concentration of the PI4K inhibitor,
- controlling and/or correcting the concentration of electrolytes,
- controlling and/or correcting the pH,
- removing waste products,
- maintaining the hematocrit level in the physiological range, particularly between 25 and 35%.

In certain embodiments, the perfusate undergoes dialysis during the perfusion step for
- controlling and/or correcting the concentration of electrolytes,
- controlling and/or correcting the pH,
- removing waste products,
- maintaining the hematocrit level in the physiological range, particularly between 25 and 35%.

The perfusate may further comprise additives such as insulin to adjust glucose concentration, a vasodilator to maintain a constant flow of the perfusate though the vessels of the liver graft, bile acids for bile stimulation, an anticoagulant to prevent prevent blood clotting, antibiotics to prevent the growth of bacteria or glucocorticoids that have antiinflammatory and immunosuppressive effects.

In certain embodiment, the perfusate further comprises one or more additives selected from insulin, a vasodilator, a bile acid, an anticoagulant, an antibiotic and a steroid.

Insulin may be administered with a concentration of 0.45 IU/mL. The amount of infused insulin is automatically controlled by a controller based on real-time glucose measurements to keep glucose concentration between 3 and 9 mmol/L.

In certain embodiments, insulin is added to the perfusate.

In certain embodiments, insulin is added to the perfusate in such an amount that a glucose concentration of 3 to 9 mM, particularly 7 mM, is maintained.

A vasodilator such as epoprostenol may be administered with a concentration of 4 mg/mL. Similar to insulin, the total amount of added vasodilator is automatically controlled by measuring the flow in the hepatic artery. When flow drops below 0.25 L/min, the infusion is ramped up until the flow is stable at 0.25 L/min.

In certain embodiments, a vasodilator is added to the perfusate.

In certain embodiments, a vasodilator is added to the perfusate in such an amount, that the flow in the hepatic artery is 0.25 L/min.

In certain embodiments, the vasodilator is epoprostenol.

In certain embodiments, an antibiotic is added to the perfusate.

In certain embodiments, 1.5 to 3.5 g, particularly 2.25 g, antibiotic per 2500 ml perfusate per 24 h is added to the perfusate during the perfusion step.

In certain embodiments, the antibiotic is Tazobactam.

In certain embodiments, a steroid is added to the perfusate.

In certain embodiments, a bolus of 300 to 1000mg, particularly 0.5 g, steroid per 2500 ml perfusate per 24 h is added during the perfusion step.

In certain embodiments, the steroid is a glucocordicoid.

In certain embodiments, the steroid is Methylprednisolone.

In certain embodiments, a bile acid is added to the perfusate.

In certain embodiments, 50 to 100 mg/h, particularly 70mg/h bile acid per 2500 ml perfusate is added during the perfusion step.

In certain embodiments, the bile acid is ursodesoxycholic acid or taurocholic acid.

In certain embodiments, an anticoagulant is added to the perfusate.

In certain embodiments, 1000 to 1500 U, particularly 1000 U anticoagulant per hour is added during the perfusion step.

In certain embodiments, the anticoagulant is heparin.

At the beginning of the perfusion step, additives may not be added continuously but an initial concentration is achieved by adding an initial bolus to the perfusion medium.

In certain embodiments, the perfusion medium comprises
- an initial bolus of 1.5 to 3.5 g, particularly 2.25 g antibiotic per 2500 ml perfusion medium, and/or
- an initial bolus of 300mg to 1000mg, particularly 0.5 g steroid per 2500 ml perfusion medium.

In certain embodiments, the perfusion medium comprises
- an initial bolus of 1.5 to 3.5 g, particularly 2.25 g Tazobactam per 2500 ml perfusion medium, and/or
- an initial bolus of 300mg to 1000mg, particularly 0.5 g Methylprednisolone per 2500 ml perfusion medium.

Maximum activity of mammalian enzymes including hepatic enzymes such as ATGL is typically achieved under normothermic conditions, i.e. at a temperature about 37 °C. Thus, it is beneficial to perform the perfusion step under normothermic conditions. The perfusion step may therefore also be referred to as normothermic perfusion step.

In certain embodiments, the perfusion step is performed at 34 to 37°C, particularly at 37°C.

In certain embodiments, perfusion is performed at the following conditions
- 70-90 mmHg systolic and 40-60 mmHg diastolic hepatic arterial pressure (0.25 L/min average arterial flow),
- 2-10 mmHg portal venous pressure (1 L/min average portal venous flow),
- 12 kPa partial pressure of oxygen, with >98% oxygen saturation
- 65% oxygen saturation in the vena cava,
- 0 mmHg constant pressure in the vena cava.

In certain embodiments, the method further comprises a preparation step comprising
- closing the infrahepatic vena cava, particularly by suturing, and/or
- cannulating the hepatic artery, the portal vein, the suprahepatic vena cava and the common bile duct.

Before the normothermic perfusion as described herein is performed, the liver may be flushed to cool down the organ and therefore slow down the metabolism. Secondly, blood cells shall be flushed out and removed to prevent coagulation issues. A flushing step is usually performed in every standard transplantation procedure. It may for instance not only be performed when the liver is immediately perfused as described herein but also in cases where the liver is not immediately connected to a normothermic perfusion machine but first transported to another hospital where normothermic perfusion and subsequent liver transplantation are performed.

In contrast to perfusion, where the perfusate circulates several times through the liver graft, the solution is only run through the organ once (i.e. flushed) when a flushing step is performed.

A first Cold flush may be performed *in-situ,* i.e. *in situ* cold flush, sometimes followed by a second *ex-situ* cold flush. *In-situ* cold flush relates to performing a cold flush while the liver is still within the donor organism (postmortal). *Ex-situ* cold flush relates to performing a cold flush outside the donor organism after the liver has been harvested from the (deceased) donor.

In certain embodiments, the method further comprises a flushing step, the flushing step comprising flushing the steatotic liver graft with a preservation solution, particularly a preservation solution selected from IGL-1 solution, UW solution, Belzer solution.

In certain embodiments, the flushing step is performed before the perfusion step.

In certain embodiments, the flushing step comprises an *in-situ* cold flush, wherein the steatotic liver graft is flushed with a preservation solution *in-situ.*

In certain embodiments, the flushing step comprises an *ex-situ* cold flush, wherein the steatotic liver graft is flushed with a preservation solution *ex-situ.*

In certain embodiments, the flushing step comprises an *in-situ* cold flush, wherein the steatotic liver graft is flushed with a preservation solution *in-situ,* followed by an *ex-situ* cold flush, wherein the steatotic liver graft is flushed with a preservation solution *ex-situ.*

In certain embodiments, the preservation solution is selected from IGL-1 solution, UW solution, Belzer solution.

After organ procurement and in-situ and/or ex-situ cold flush, the graft may be preserved on ice i.e. static cold storage (SCS) until it is connected to a normothermic perfusion device.

In certain embodiments, the method further comprises a static cold storage step, wherein the liver is stored on ice.

In certain embodiments, the static cold storage step is performed after the flushing step and before the perfusion step.

Before performing the perfusion step as described above, i.e. normothermic perfusion, performing a hypothermic oxygenated perfusion instead of static cold storage may be beneficial for the treatment. Hypothermic oxygenated perfusion has protective effects on the graft with respect to ischemic reperfusion injury. So, hypothermic oxygenated perfusion treatment may be beneficial to keep the organ in a better shape before the actual treatment is started on the normothermic perfusion machine.

In certain embodiments, the method further comprises a hypothermic oxygenated perfusion step, the hypothermic oxygenated perfusion step comprising perfusing the steatotic liver graft with a hypothermic machine perfusion solution.

In certain embodiments, the hypothermic oxygenated perfusion step is performed after the flushing step and before the perfusion step.

In certain embodiments, the hypothermic machine perfusion solution is Belzer MPS solution.

During the hypothermic oxygenated perfusion step, oxygen is added via a membrane oxygenator to achieve an oxygen partial pressure of > 100 kPa. Oxygen saturation of the perfusate is 100%. Oxygen flow to the oxygenator is at least 0.5 L/min, particularly 0.5 to 1 L/min.

In certain embodiments, the hypothermic oxygenated perfusion step is performed at > 100 kPa partial oxygen pressure.

The hypothermic oxygenated perfusion is typically performed on ice, i.e. ideally at 4°C.

In certain embodiments, the hypothermic oxygenated perfusion step is performed at 0 to 12°C, particularly 8°C.

In certain embodiments, the hypothermic oxygenated perfusion step is performed at 4 to 8°C, particularly at 4°C.

In certain embodiments, the hypothermic oxygenated perfusion step is performed for 1 to 8 hours, particularly for 2 h.

In certain embodiments, the compound is selected from a PI4KB inhibitor of formula 1a, 2a, and 3, 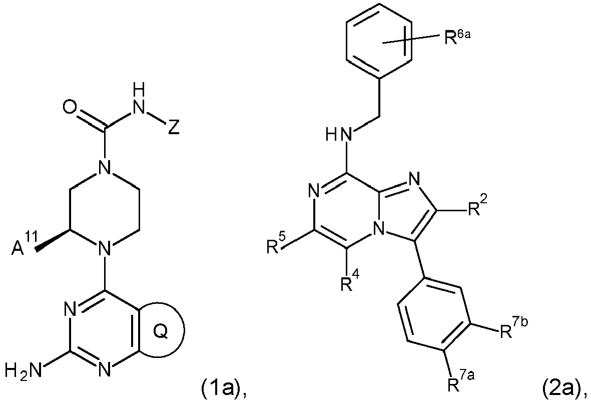 wherein Z, A¹¹, Q, R², R4, R⁵, R^{6a}, R^{7a}, R^{7b} and c are defined as described above.

In certain embodiments, the compound is a PI4KB inhibitor of formula 1a.

In certain embodiments, A¹¹ is methyl, ethyl, hydroxymethyl or hydroxyethyl.

In certain embodiments, A¹¹ is methyl or ethyl.

In certain embodiments, A¹¹ is methyl.

In certain embodiments, A¹¹ is H, methyl or ethyl.

In certain embodiments, A¹¹ is H or methyl.

In certain embodiments, Z is selected from a moiety of formula Za, Zb, Zc, Zd, Ze, Zf, Zg, Zh and Zi,

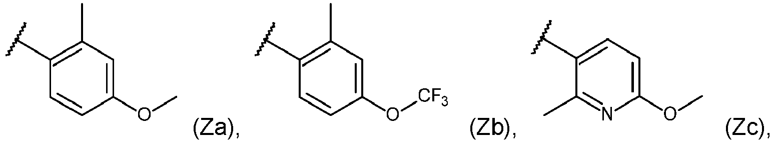

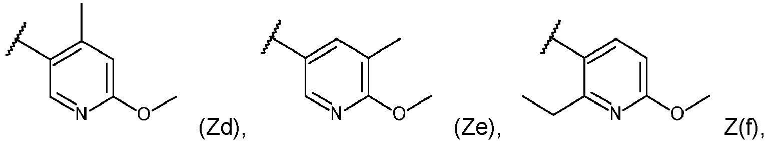

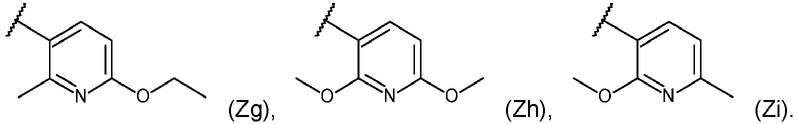

In certain embodiments, Z is selected from a moiety of formula Za, Zc and Zd.

In certain embodiments, Z is a moiety of formula Za.

In certain embodiments, one of X and Y is N and the other is C(H).

In certain embodiments, X is C(H) and Y is N.

In certain embodiments, Q is selected from a heterocycle of formula Qa, Qb, Qc, Qd and Qe,

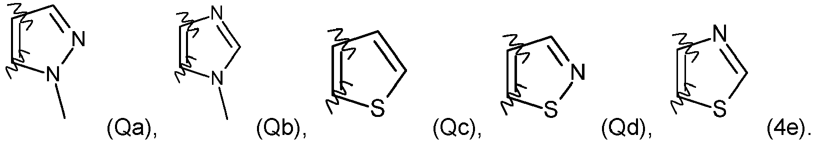

In certain embodiments, Q is selected from a heterocycle of formula Qa and Qb.

In certain embodiments, Q is a heterocycle of formula Qa.

In certain embodiments, A¹¹ is methyl and Q is selected from a heterocycle of formula Qa and Qb.

In certain embodiments, A¹¹ is methyl and Q is a heterocycle of formula Qa.

In certain embodiments, Q is selected from a heterocycle of formula Qa and Qb and Z is selected from a moiety of formula Za, Zc and Zd.

In certain embodiments, Q is a heterocycle of formula Qa and Z is selected from a moiety of formula Za, Zc and Zd.

In certain embodiments, A¹¹ is methyl, Q is selected from a heterocycle of formula Qa and Qb and Z is selected from a moiety of formula Za, Zc and Zd.

In certain embodiments, A¹¹ is methyl, Q is a heterocycle of formula Qa and Z is selected from a moiety of formula Za, Zc and Zd.

In certain embodiments, Q is selected from a heterocycle of formula Qa and Qb and Z is a moiety of formula Za.

In certain embodiments, Q is a heterocycle of formula Qa and Z is a moiety of formula Za.

In certain embodiments, A¹¹ is methyl, Q is selected from a heterocycle of formula Qa and Qb and Z is a moiety of formula Za.

In certain embodiments, A¹¹ is methyl, Q is a heterocycle of formula Qa and Z is moiety of formula Za.

In certain embodiments, R², R⁴ and R⁵ are independently selected from H or methyl.

In certain embodiments, R² is methyl, and R⁴ and R⁵ are H.

In certain embodiments, R^{6a} is So₂R⁸, wherein R⁸ is a C₁-C₆ alkyl.

In certain embodiments, R^{6a} is So₂R⁸, wherein R⁸ is CH₃.

In certain embodiments, R^{7a} is selected from OH, NH₂ and OCH₃.

In certain embodiments c is 1.

In certain embodiments, R⁸ and R⁹ are independently selected from a C₁-C₆ alkyl.

In certain embodiments, R⁸ and R⁹ are methyl.

In certain embodiments, R^{7b} is selected from OH, halogen, NO₂, C₁-C₆ alkyl, C₁-C₆ alkoxy, C₁-C₆ alkyl-OH, NH₂, NHSO₂R⁹ and NHCOR⁸ with R⁹ and R⁸ being defined as described above.

In certain embodiments, R^{7b} is selected from OH, halogen, NO₂, C₁-C₆ alkyl, C₁-C₆ alkoxy, C₁-C₆ alkyl-OH, NH₂, NHSO₂R⁹ and NHCOR⁸ with R⁹ and R⁸ being independently selected form a C₁-C₆ alkyl.

In certain embodiments, R^{7b} is selected from OH, halogen, NO₂, C₁-C₆ alkyl, C₁-C₆ alkoxy, C₁-C₆ alkyl-OH, NH₂, NHSO₂R⁹ and NHCOR⁸ with R⁹ and R⁸ being methyl.

In certain embodiments, R^{7b} is selected from OH, halogen, NO₂, OCH₃, CF₃, OCF₃, CH₃, CH₂-OH and NHSO₂R⁸ with R⁸ being defined as described above.

In certain embodiments, R^{7b} is selected from OH, halogen, NO₂, OCH₃, CF₃, OCF₃, CH₃, CH₂-OH and NHSO₂R⁸ with R⁸ being a C₁-C₆ alkyl.

In certain embodiments, R^{7b} is selected from OH, halogen, NO₂, OCH₃, CF₃, OCF₃, CH₃, CH₂-OH and NHSO₂R⁸ with R⁸ being mehtyl.

In certain embodiments, R^{7b} is a halogen.

In certain embodiments, R^{7b} is -F.

In certain embodiments, the compound is selected from a PI4KB inhibitor of formula 1b, 2b and 3,

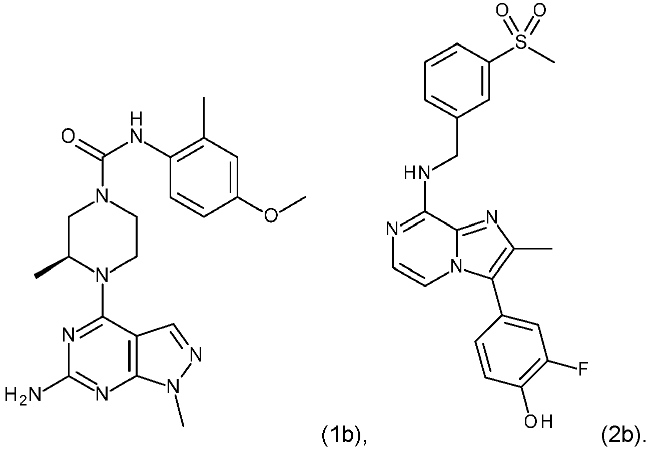

In certain embodiments, the PI4KB inhibitor is a compound of formula 1b.

The invention is further illustrated by the following examples and figures, from which further embodiments and advantages can be drawn. These examples are meant to illustrate the invention but not to limit its scope.

### Description of the Figures

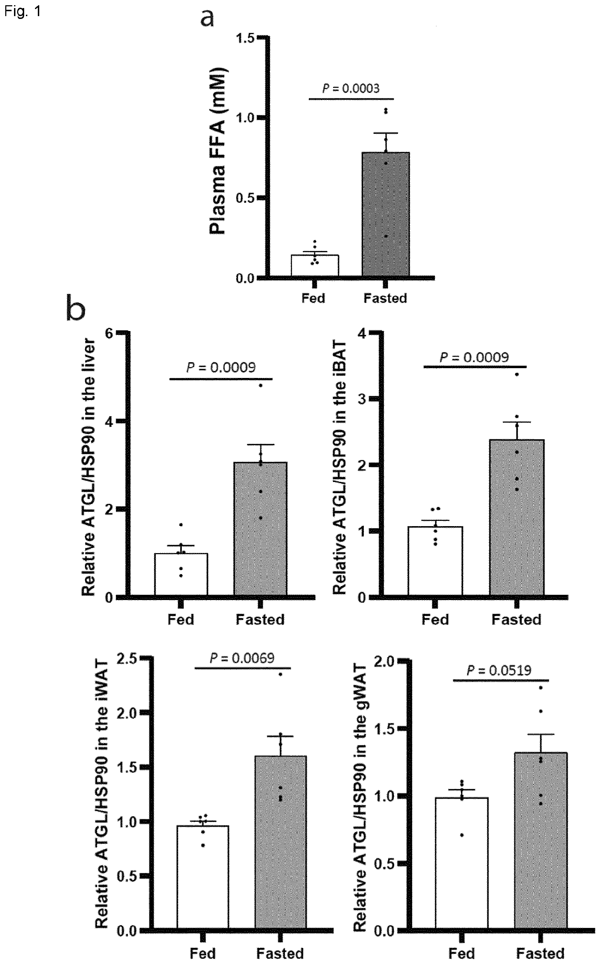

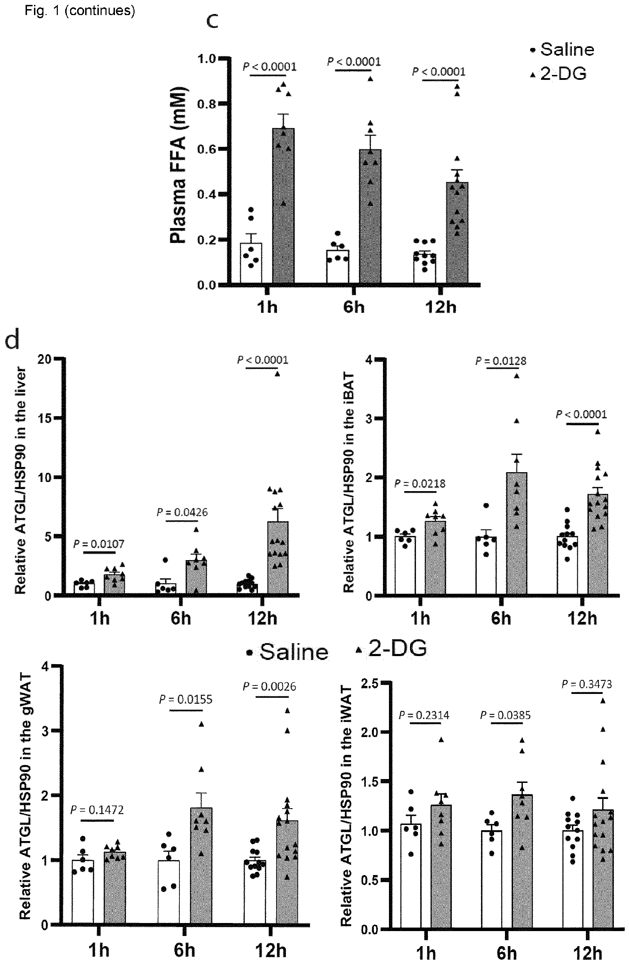

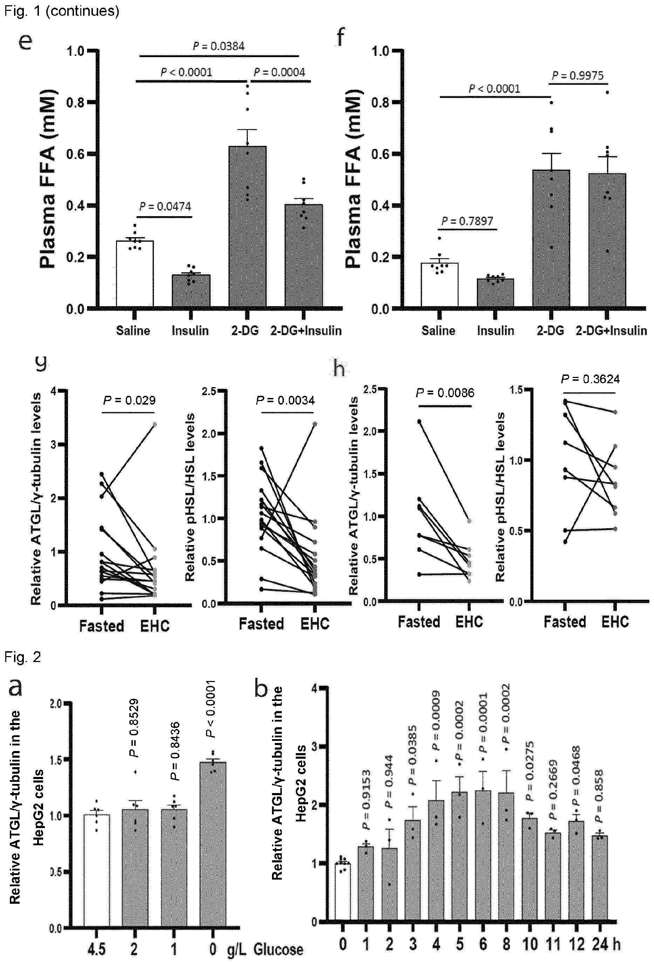

- Fig. 1: shows glucose deprivation increases lipolysis via ATGL stabilization in the adipose depots and liver. (a) Plasma FFA levels in the wild-type mice 24 h after fasting (n = 6 for both groups). (b) Quantification of ATGL levels in the lysate of liver and adipose depots from fed and fasted mice (n = 6 for both groups). (c) Plasma FFA levels in the wild-type mice 1 h, 6 h and 12 h after 2-DG (2 g/kg BW) administration (1 h and 6 h, n = 6 for saline and 8 for 2-DG group; 12 h, n = 10 for saline and 13 for 2-DG group). (d) Quantification of ATGL levels in the lysate of liver and adipose depots collected 1 h, 6 h and 12 h after 2-DG (2 g/kg BW) administration (1 h and 6 h, n = 6 for saline and 8 for 2-DG group; 12 h, n = 12 for saline and 15 for 2-DG group). (e) Plasma FFA levels in the wild-type mice after 2-DG (2 g/kg BW) treatment for 5.5 h, followed by insulin (0.75 U/kg BW) administration for 30 minutes (n = 8 for all groups). (f) Plasma FFA levels in the wild-type mice 6 h after co-administration of 2-DG (2 g/kg BW) and insulin (0.75 U/kg BW) (n = 8 for all groups). (g) Quantification of ATGL and HSLSer660ph levels in the subcutaneous adipose tissue taken before and after EHC from patients with insulin sensitivity (n = 16 patients). (h) Quantification of ATGL and HSLSer660ph levels in the subcutaneous adipose tissue taken before and after EHC from patients with insulin resistance (n = 8 patients). Data were presented as mean ± s.e.m. and analyzed using two-tailed unpaired t-test (a, b, c and d (1 h in liver, iBAT, 1 h and 6 h in iWAT, and 1 h and 6 h in gWAT)), paired t test (h), Mann-Whitney test (d (6 h and 12 h in liver, 12 h in iWAT and 12 h in gWAT)), Wilcoxon test (g) and ANOVA method with Tukey correction (e and f).

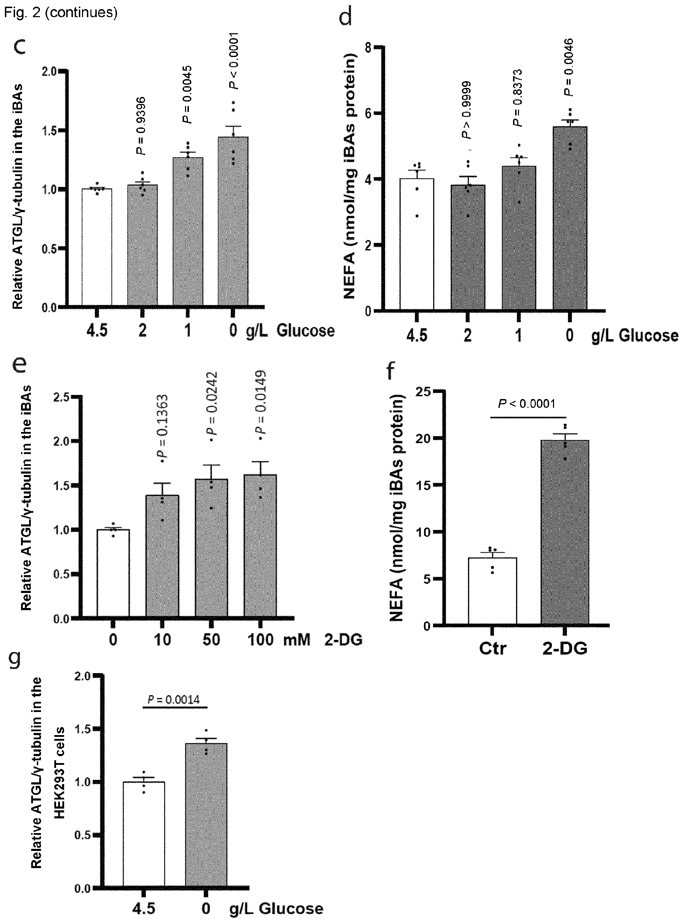

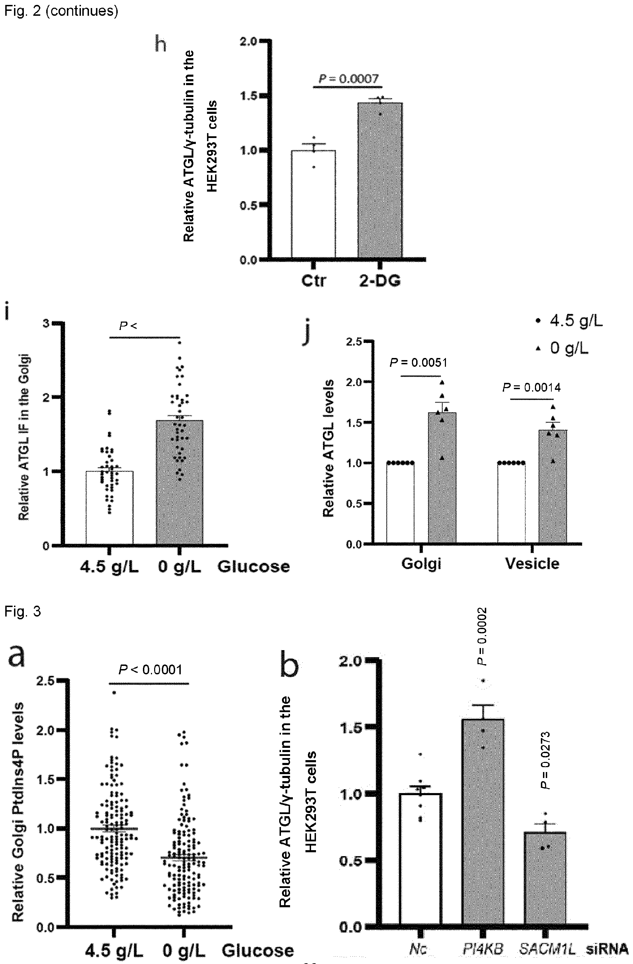

- Fig. 2: shows glucose deprivation modulates lipolysis via stabilizing ATGL in the Golgi. (a) Quantification (based on immunoblot) of ATGL in the HepG2 cell type cultured at different glucose concentrations for 6 h (n = 6 for all groups). (b) Quantification of ATGL via immunoblot in the HepG2 cell type treated with 50 mM 2-DG and harvested at indicated timepoints (n = 9 for control and 3 for all 2-DG groups). (c) Quantification (via immunoblot) of ATGL in the iBAs cultured at different glucose concentrations for 24 h (n = 6 for all groups). (d) Levels of non-esterified fatty acids (NEFAs) in the starvation medium released by iBAs in the basal state 24 h after treatment with medium at different glucose concentrations (n = 6 for all groups). (e) Quantification (via immunoblot) of ATGL in the iBAs treated with 2-DG at indicated concentrations for 24 h (n = 4 for all groups). (f) Levels of NEFAs in the starvation medium released by iBAs in the basal state 24 h after treatment with 100 mM 2-DG (n = 5 for both groups). (g) Quantification (via immunoblot) of ATGL in the HEK293T cell type 6 h after treatment with glucose free medium (n = 4 for both groups). (h) Quantification (via immunoblot) of ATGL in the HEK293T cell type 12 h after treatment with 50 mM 2-DG (n = 4 for both groups). (i) Quantification of the immunofluorescence of ATGL in the HEK293-AAV cell type 6 h after treatment with glucose free medium (43 cells used in both groups). (j) Quantification (via immunoblot) of ATGL in the whole-cell extract (WCE), Golgi fraction and vesicle fraction extracted from HEK293T cell type 6 h after treatment with glucose free medium (n = 6 for both groups). Results are shown as mean ± s.e.m. and analyzed using two-tailed unpaired *t*-test (f to i), paired t-test (j), ANOVA method with Dunnett correction for multiple comparisons between control and other groups (a, b, c and e) and Kruskal-Wallis test with Dunn's correction for multiple comparisons between control and other groups (d).

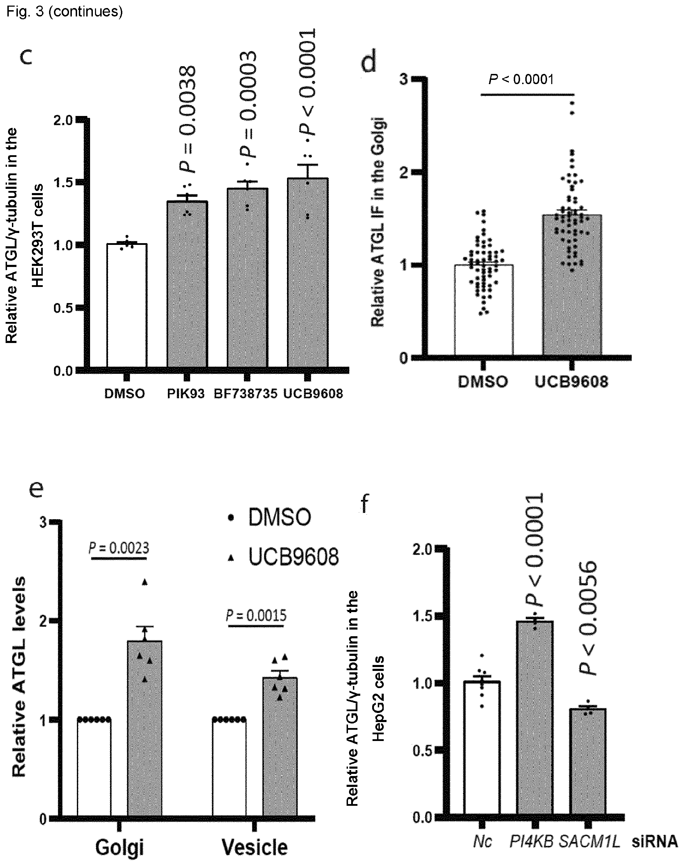

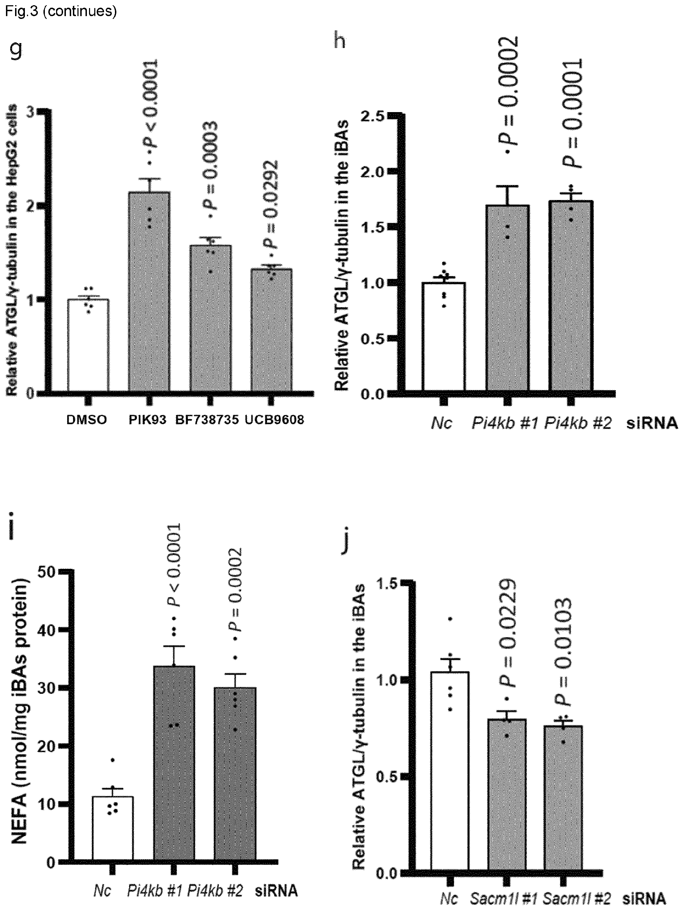

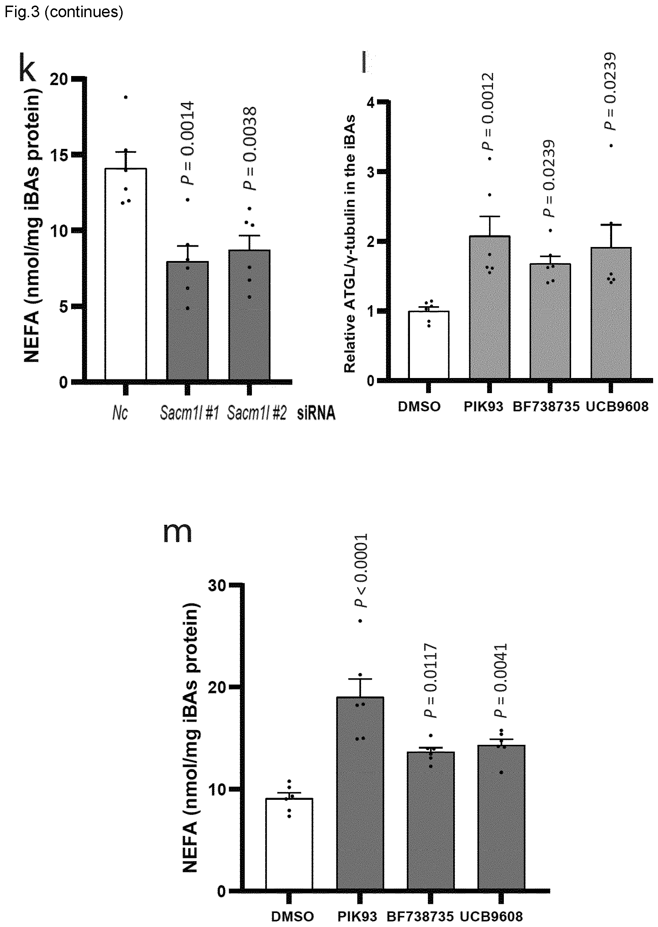

- Fig. 3: shows Golgi Ptdlns4P regulates ATGL protein stability in the Golgi apparatus. (a) Quantification of Ptdlns4P levels via immunostaining in the HEK293-AAV cell type after treatment with glucose free medium (150 cells used in both groups). (b) Quantification (via immunoblot) of ATGL in the HEK293T cell type 72 h after *PI4KB* and *SACM1L knockdown* (n = 8 for control and 4 for the other groups). (c) Quantification (via immunoblot) of ATGL in the HEK293T cell type 24 h after treatment with 5 µM PIK93, 10 µM BF738735 and 10 µM UCB9608 (n = 6 for all groups). (d) Quantification of the immunofluorescence of ATGL in the HEK293-AAV cell type 24 h after treatment with UCB9608 (61 cells used in both groups). (e) Quantification (via immunoblot) of ATGL in the WCE, Golgi fraction and vesicle fraction extracted from HEK293T cell type 6 h after treatment with UCB9608 (n = 6 for both groups). (f) Quantification (via immunoblot) of ATGL in the HepG2 cell type 72 h after *PI4KB* and *SACM1L knockdown* (n = 8 for control and 4 for the other groups). (g) Quantification (via immunoblot) of ATGL in the HepG2 cell type 24 h after treatment with 5 µM PIK93, 10 µM BF738735 and 10 µM UCB9608 (n = 6 for all groups). (h) Quantification (via immunoblot) of ATGL in the iBAs 72 h after *Pi4kb* knockdown (n = 8 for control and 4 for the other groups). (i) Levels of NEFAs in the starvation medium released by iBAs in the basal state 72 h after *Pi4kb* knockdown (n = 6 for all groups). (j) Quantification (via immunoblot) of ATGL in the iBAs 72 h after *Sacm1l* knockdown (n = 6 for control and 4 for the other groups). (k) Levels of NEFAs in the starvation medium released by iBAs in the basal state 72 h after *Sacm1l* knockdown (n = 6 for all groups). (I) Quantification (via immunoblot) of ATGL in the iBAs 24 h after treatment with 1 µM PIK93, 1 µM BF738735 and 1 µM UCB9608 (n = 6 for all groups). (m) Levels of NEFAs in the starvation medium released by iBAs in the basal state 24 h after treatment with 1 µM PIK93, 1 µM BF738735 and 1 µM UCB9608 (n = 6 for all groups). Data were presented as mean ± s.e.m. and analyzed using paired *tl*st (e), Mann-Whitney test (a and d), ANOVA method with Dunnett correction for multiple comparisons between control and other groups (b, c, f-k and m) and Kruskal-Wallis test with Dunn's correction for multiple comparisons between control and other groups (I).

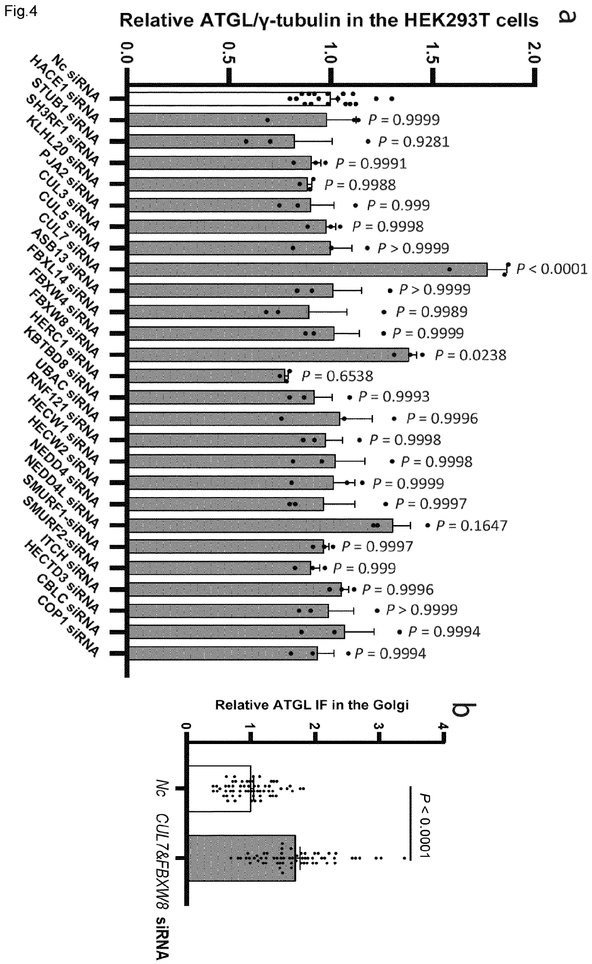

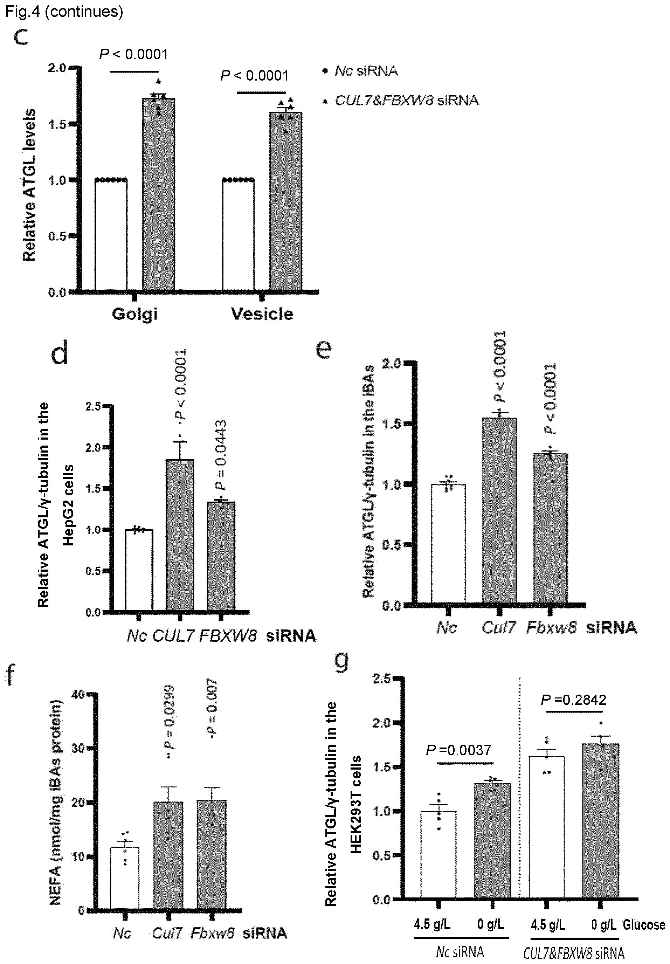

- Fig. 4: shows the Golgi resident E3 ligase complex CUL7FBXW8 poly-ubiquitylates ATGL for degradation. (a) Quantification of ATGL protein levels of HEK293T WCE in a small-scale siRNA screen via immunoblot (n = 18 for control and 3 for the other groups). (b) Photomicrographs of the immunofluorescence of ATGL in the HEK293-AAV cell type 72 h after *CUL7&FBXW8* knockdown (61 cells used in both groups). Scale bar, 5 µm. (c) Immunoblot of ATGL in the WCE, Golgi fraction and vesicle fraction extracted from HEK293T cell type 72 h after *CUL7&FBXW8* knockdown (n = 6 for both groups). Asterisk * indicates non-specific band. (d) Immunoblot of ATGL in the HepG2 cell type 72 h after *CUL7* and *FBXW8* knockdown (n = 8 for control and 4 for the other groups). (e) Immunoblot of ATGL in the iBAs 72 h after *Cul7* and *Fbxw8* knockdown (n = 8 for control and 4 for the other groups). (f) Levels of NEFAs in the starvation medium released by iBAs in the basal state 72 h after knockdown of *Cul7* and *Fbxw8* (n = 6 for all groups). (g) Immunoblot of ATGL in the HEK293T cell type 6 h after treatment with glucose free medium in the absence of CUL7FBXW8 (n = 5 for all groups). Data were presented as mean ± s.e.m. and analyzed using two-tailed unpaired t-test (b and g), paired t-test (c), ANOVA method with Dunnett correction for multiple comparisons between control and other groups (a, d and e) and Kruskal-Wallis test with Dunn's correction for multiple comparisons between control and other groups (f).
- Fig. 5: shows recruitment of FBXW8 to the Golgi via interaction with Ptdlns4P. (a-b) Quantification (via immunoblot) of CUL7 and FBXW8 in the WCE, Golgi fraction and vesicle fraction extracted from HEK293T cell type after treatment with glucose free medium (a) or UCB9608 (b) (n = 6 for all groups). Asterisk * indicates non-specific band. (c) Blotting analysis of PIP strips incubated with recombinant proteins FBXW8-FLAG and FBXW8 mutant-FLAG. (d-e) Quantification (via immunoblot) of FBXW8-FLAG (d) and FBXW8 mutant-FLAG (e) in the WCE, Golgi fraction and vesicle fraction extracted from HEK293T cell type after treatment with glucose free medium (n = 6 for both groups). (f) Schematic diagram illustrating the working model of the mechanism by which glucose availability is coupled to lipolysis via Golgi Ptdlns4P. Results are shown as mean ± s.e.m. and analyzed using paired *t*-test (a, b, d and e).
- Fig. 6: shows regulation of ATGL-driven lipolysis and fatty liver development via manipulating Golgi Ptdlns4P- CUL7FBXW8-ATGL axis. (a) Quantification (via immunoblot) of ATGL protein in the liver from hepatocyte specific *Pi4kb* knockout mice 2 weeks after AAV8 administration (n = 8 for both groups). (b) Quantification (via immunoblot) of ATGL protein in the liver from hepatocyte specific *Cul7* and *Fbxw8* knockout mice 2 weeks after AAV8 administration (n = 6 for all groups). (c) Quantification (via immunoblot) of ATGL protein in the liver 1 h after 2-DG injection under the background of *Cul7* and *Fbxw8* double knockout in the hepatocytes (n = 6 for all groups). (d) Determination of hepatic TG levels in NCD feeding mice 4 weeks after AAV8 administration or HFD feeding mice 8 weeks after AAV8 administration (n = 10 for both groups in NCD and 5 for both groups in HFD). (e) Determination of hepatic TG levels in hepatocyte specific *Cul7* and *Fbxw8* knockout mice fed in NCD and HFD after AAV8 administration (n = 11 for all groups in NCD and 5 for all groups in HFD). (f) Schematic illustration of experimental design to administer UCB9608 to wild-type mice. (g) Plasma FFA levels in wild-type mice 8 weeks after administration of DMSO and UCB9608 (n = 12 for both groups). (h) Hepatic TG levels in DMSO- and UCB9608-treated mice (n = 15 for both groups). Data were presented as mean ± s.e.m. and analyzed using two-tailed unpaired *t*-test (a, c, d and h), Mann-Whitney test (g) and ANOVA method with Dunnett correction for multiple comparisons between control and other groups (b and e).

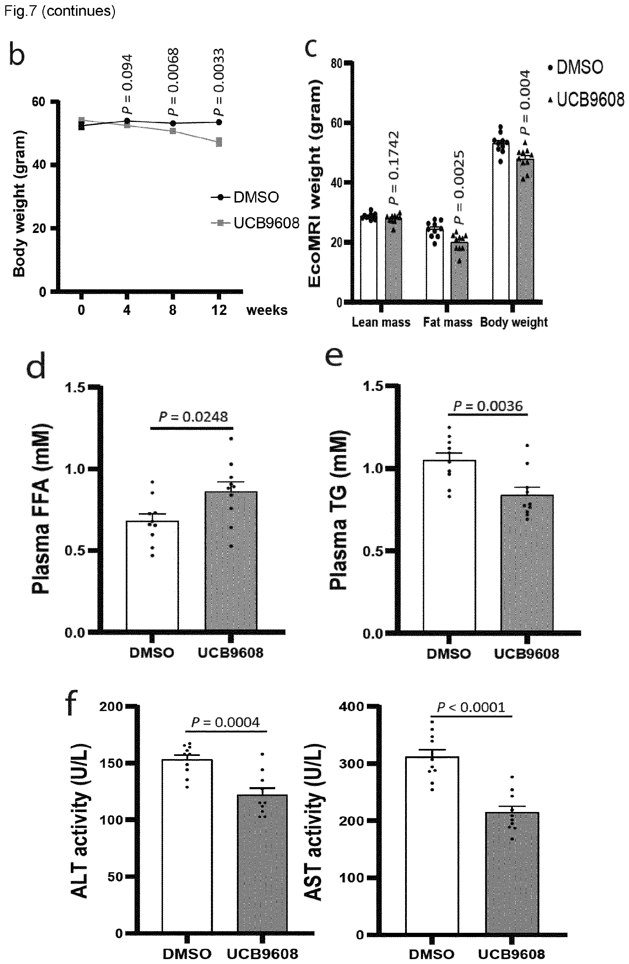

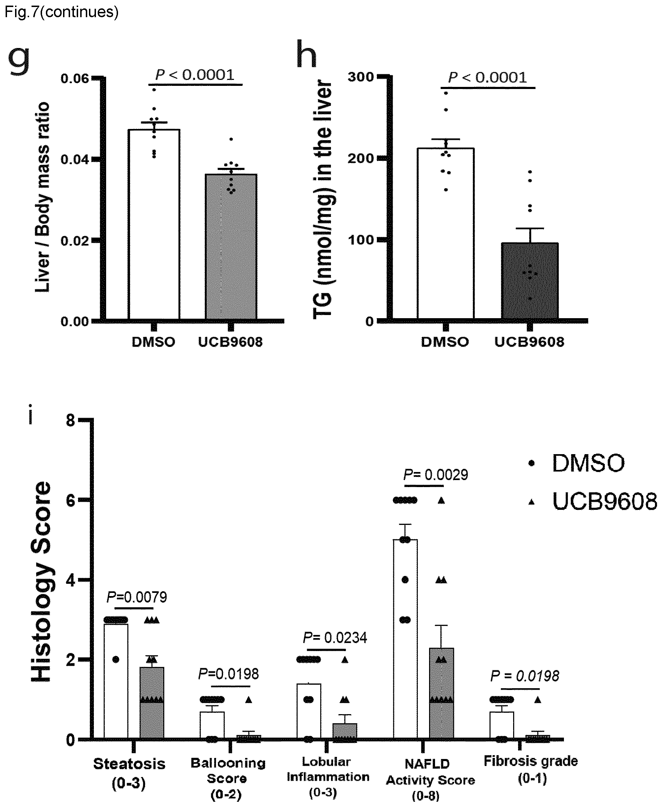

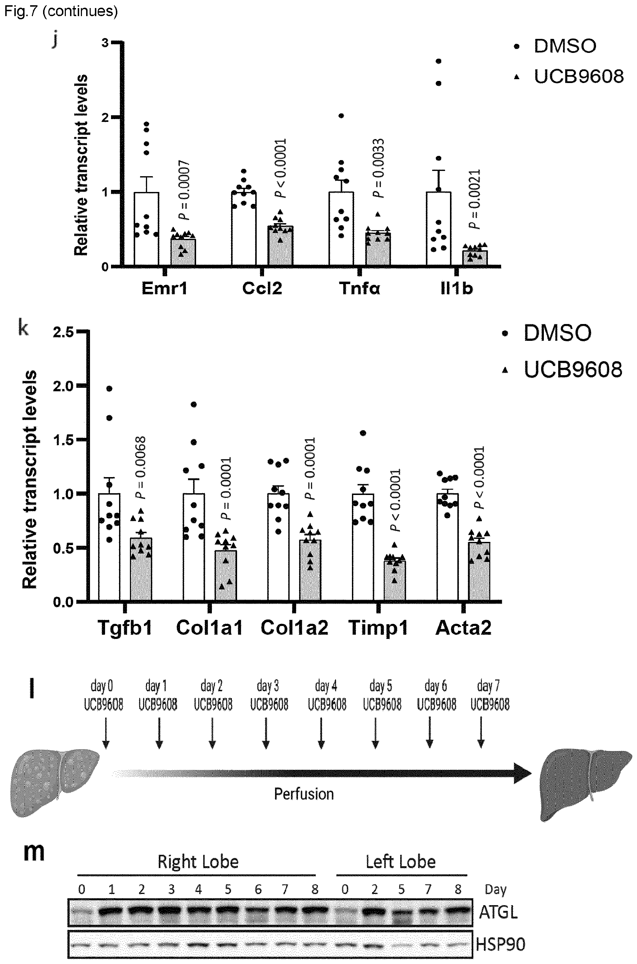

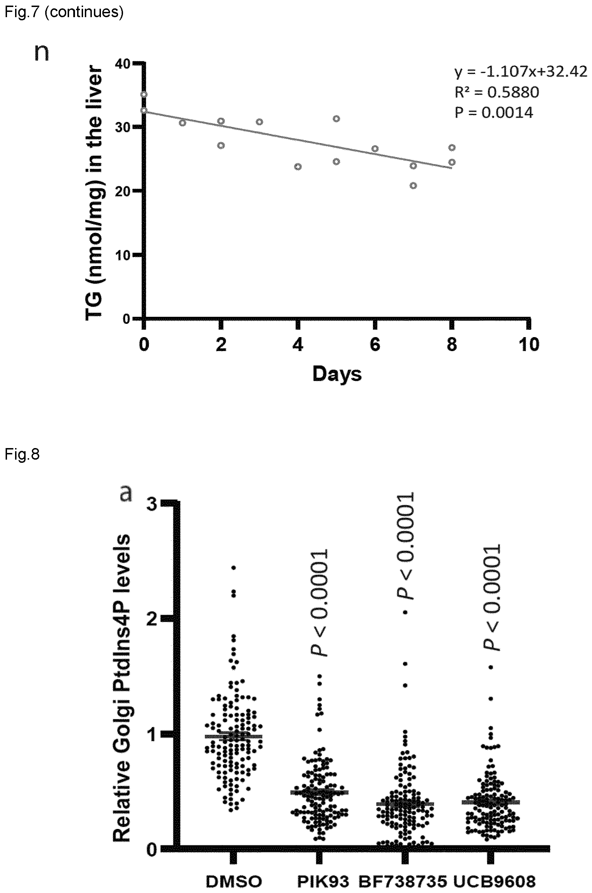

- Fig. 7: shows amelioration of murine NASH progression and steatosis in human liver graft via blocking Golgi Ptdlns4P generation, (a) Schematic illustration of experiment design to administer UCB9608 to wild-type mice after induction of NASH via HFD, high glucose/fructose containing water and thermoneutral housing. (b) Body weight gain during the period of UCB9608 treatment (n = 10 for both groups). (c) EcoMRI analyses of lean mass, fat mass and body weight to the NASH mice (n = 10 for both groups). (d) Plasma FFA levels 12 weeks after administration of UCB9608 (n = 10 for both groups). (e) Plasma TG levels 12 weeks after administration of UCB9608 (n = 10 for both groups). (f) ALT and AST activity in the plasma from NASH mice after administration of UCB9608 (n = 10 for both groups). (g) Liver mass/body weight ratio of NASH mice after administration of UCB9608 (n = 10 for both groups). (h) Determination of hepatic TG levels of NASH mice after administration of UCB9608 (n = 10 for both groups). ( (i) Histology score of livers from NASH mice treated by DMSO and UCB9608 (n = 10 for both groups). (j) Transcript levels of inflammatory markers in NASH livers as determined by qPCR (n = 10 for both groups). (k) Transcript levels of fibrosis markers in NASH livers as determined by qPCR (n = 10 for both groups). (l) Schematic illustration of experiment design to administer UCB9608 to a human liver graft ex *vivo* during normothermic machine perfusion. (m-n) Quantification (via immunoblot) of ATGL protein and quantification of TG levels in the human liver biopsies taken at different time points from both lobes. Results are shown as mean ± s.e.m. and analyzed using two-tailed unpaired t-test (b to g, j (Ccl2 and Tnfα) and k (Col1a2, Timp1 and Acta2)), Mann-Whitney test (h, i, j (Emr1and II1b) and I (Tgfb1 and Col1a1)) and Pearson's correlation (n).

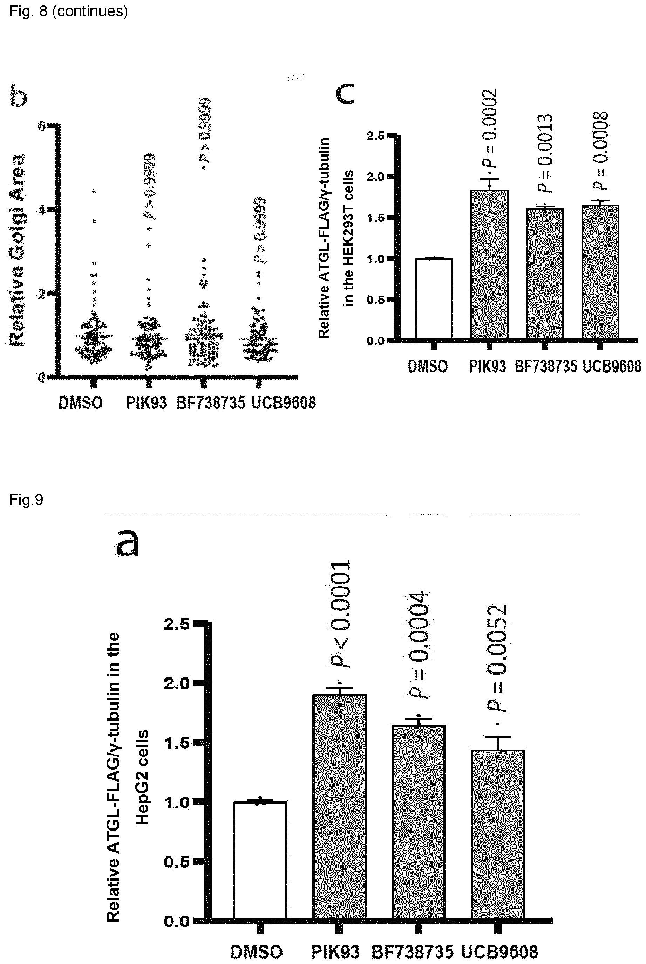

- Fig. 8: shows Golgi Ptdlns4P regulates ATGL protein stability in the Golgi apparatus. (a) Quantification of Ptdlns4P levels via immunostaining in the HEK293-AAV cell type after 24 h treatment with 5 µM PIK93, 10 µM BF738735 and 10 µM UCB9608 (136 cells used in all groups). (b) Quantification of Golgi area via anti-GM130 immunostaining in the HEK293-AAV cell type 72 h treatment with 5 µM PIK93, 10 µM BF738735 and 10 µM UCB9608 (100 cells used in all groups). (c) Quantification (via immunoblot) of ectopically expressed ATGL in the HEK293T cell type 24 h after treatment with 5 µM PIK93, 10 µM BF738735 and 10 µM UCB9608 (n = 3 for all groups). Results are shown as mean ± s.e.m. and analyzed ANOVA method with Dunnett correction for multiple comparisons between control and other groups (c) and Kruskal-Wallis test with Dunn's correction for multiple comparisons between control and other groups (a and b).
- Fig. 9: shows glucose deprivation elevates ATGL protein in the Golgi via Ptdlns4P regulation. (a) Quantification (via immunoblot) of ectopically expressed ATGL in the HepG2 cell type 24 h after treatment with 5 µM PIK93, 10 µM BF738735 and 10 µM UCB9608 (n = 3 for all groups). Results are shown as mean ± s.e.m. and analyzed using ANOVA method with Dunnett correction for multiple comparisons between control and other groups (a).

### Examples

### Example 1: Glucose deprivation elevates ATGL-driven lipolysis levels

Previous work demonstrated that intracellular FAs modulate ATGL-driven lipolysis via peroxisomal FA oxidation, thus linking FA availability to FA liberation. To elucidate whether other general cell-autonomous mechanisms exist that modulate lipolysis in response to the availability of other nutrients, such as glucose, mice were fasted for 24 hours and the levels of plasma free fatty acids (FFAs), a surrogate marker of lipolysis, were quantified. The inventors observed that FFA levels were significantly upregulated upon fasting (Fig. 1a). Next, the levels of lipolytic proteins were analyzed, including ATGL, phosphorylated HSL, HSL, MGL, perilipin 1 (PLIN1) and comparative gene identification-58 (CGI-58), in the liver and adipose depots from fasted mice. Interestingly, only ATGL exhibited significantly increased protein levels in the interscapular brown adipose tissue (iBAT), inguinal white adipose tissue (iWAT) and liver in response to long-term fasting (Fig. 1b), suggesting that a general sensory system adjusts ATGL-driven lipolysis levels to meet the cellular energy demands. Both iWAT and gonadal white adipose tissue (gWAT) displayed slightly upregulated pHSL levels, suggesting a certain level of altered hormone signaling. To understand the timeline of HSL phosphorylation and ATGL upregulation in response to long-term glucose deprivation, mice were treated with 2-Deoxy-D-glucose (2-DG), a non-hydrolysable glucose analog that effectively blocks glucose utilization and thus mimics glucose deprivation, *in vivo.* Lipolysis and lipolytic protein levels were analyzed at different time points after 2-DG treatment. Similar to fasting, glucose deprivation significantly induced lipolysis rapidly after 2-DG treatment (Fig. 1c), suggesting the molecular pathways involved may not rely on transcriptional regulation. To analyze the hormonal axis, the inventors first determined levels of plasma insulin and only observed reduction of insulin levels 1 hour after 2-DG administration, suggesting only a partial involvement of insulin. Thus, the inventors next quantified the main players involved in lipolysis in both liver and adipose depots at different time points. Intriguingly, upregulation of HSL phosphorylation was only observed in white adipose tissues 1 hour after 2-DG administration, while ATGL protein levels were increased in the liver and iBAT after 1 hour and showed further accumulation in the liver, iBAT, iWAT and gWAT in response to longer glucose deprivation (Fig. 1d), suggesting that HSL contributes partially in this altered lipolytic response only as an immediate regulator, while ATGL regulation maintains lipolysis at high levels throughout the course of glucose deprivation via a yet unknown mechanism. Hence, the immediate effect of insulin on long-term glucose-deprived mice 6 hours after 2-DG treatment was tested. Unexpectedly, lipolysis levels were only partially suppressed and levels of pHSL and ATGL remained unchanged in the background of glucose deprivation 30 minutes after insulin treatment (Fig. 1e). Moreover, the anti-lipolytic effect of insulin was totally absent in the long-term glucose deprivation condition 6 hours after co-injection of 2-DG and insulin (Fig. 1f). These observations led the inventors to postulate the existence of cell intrinsic mechanisms of ATGL-driven lipolysis regulation via sensing intracellular glucose availability, independently of hormone signaling, which is effective in both short- and long-term glucose deprivation induced lipolysis to different extents. More importantly, the inventors obtained subcutaneous fat from patients collected before and after euglycemic hyperinsulinemic clamp (EHC), corresponding to the low and high circulated glucose states, respectively. Interestingly, higher ATGL levels were observed in subcutaneous adipose tissues acquired before EHC in patients with either insulin sensitivity or insulin resistance (Fig. 1g, h), suggesting a similar ATGL regulation occurs in humans as observed in the mouse models.

To gain a better mechanistic understanding of the ATGL regulation in these organs, the inventors examined *Atgl* transcript levels in the liver and adipose depots from fasted or glucose-deprived mice and found that *Atgl* transcript levels were unchanged in the adipose tissues, further indicating that the regulation is not dependent on transcriptional events. In contrast, long-term fasting and glucose deprivation promoted *Atgl* transcript levels in the liver, possibly due to hepatic PPARα activation in response to FA signaling in the liver. As adipose tissue is well-known to contribute to FA release into the blood plasma, it was examined whether ATGL upregulation in the adipose tissue, instead of the liver, promoted an increase in plasma FFA levels during glucose deprivation. Therefore, the inventors took advantage of liver- and adipose tissue-specific *Atgl* knockout mice (*AtglLKO* and *AtglAKO* mice, respectively) and found that only adipose tissue ATGL was essential for increased plasma FFA levels in response to glucose deprivation. Taken together, the existence of a general cell-intrinsic mechanism, which modulates ATGL-driven lipolysis via sensing glucose availability, independently of insulin signaling was demonstrated.

### Example 2: Glucose deprivation increases ATGL protein levels in the Golgi apparatus

As ATGL protein levels are predominantly regulated independently of transcription in the glucose-deprived mice, the inventors focused on the mechanism of ATGL stabilization in response to glucose availability. Given the complexity of the mouse model, the inventors first examined whether glucose deprivation-induced ATGL stabilization could be recapitulated in HepG2 cell type, an immortalized human liver cell line. In agreement with the *in vivo* data, it was observed that both glucose withdrawal from the culture medium and 2-DG treatment strongly induced ATGL protein abundance without changing transcript levels, suggesting the same post-translational regulation of ATGL as in the liver and adipose tissue (Fig. 2a, b). This process was further corroborated in immortalized brown adipocytes (iBAs), where increased lipolysis levels and ATGL protein abundance despite unchanged *Atgl* transcript levels after blocking glucose utilization were observed (Fig. 2c, d, e,f). In addition, regulation of ATGL protein was also reproduced in HEK293T and HEK293-AAV cell types (both immortalized human embryonic kidney cell types), in response to glucose deprivation (Fig. 2g, h). Moreover, the inventors deprived serum, amino acids as well as fatty acids in the culture medium and observed that only serum deprivation promoted the accumulation of intracellular ATGL levels, indicating that shortage of glucose and serum adopts the same mechanism to induce liberation of FAs from TG stores via enhanced ATGL-driven lipolysis.

After being synthesized in the ER, ATGL is transported to the Golgi, where it then translocates to the surface of LDs. Inhibition of this process strongly reduces intracellular ATGL proteins levels, highlighting the importance of the Golgi-LD axis in modulating ATGL abundance and function. Therefore, the inventors next focused on the LDs and Golgi as potential sites where ATGL protein levels might be regulated upon glucose deprivation. First, the inventors isolated LDs from HepG2 cell type and observed that ATGL levels increased in the LDs fraction upon glucose deprivation, indicating that ATGL might be regulated on the LDs. Thus, the inventors treated HepG2 cell type with inhibitors of DGAT1 and DGAT2, the key enzymes for the esterification of FAs to generate TG, to block TG production and thus to deplete LD abundance within 48 hours. Intriguingly, even in the absence of LDs, glucose deprivation could still increase endogenous ATGL. Conversely, HepG2 cell type failed to upregulate intracellular ATGL levels upon glucose deprivation after treatment with BFA (Brefeldin A), an antibiotic which blocks Golgi formation, indicating that ATGL abundance is regulated at the level of the Golgi rather than on the LD surface. Thus, the inventors quantified ATGL levels in the Golgi by immunostaining and fractionation and observed significantly elevated protein levels in the Golgi and vesicle fractions in response to glucose deprivation (Fig. 2i, j). These data demonstrate that ATGL is primarily regulated at the level of the Golgi and are subsequently translocated to vesicles and its functional site on the LD surface to modulate lipolysis.

### Example 3: Reduction of Golgi Ptdlns4P levels promotes ATGL stability

Both glucose and serum deprivation have been reported to reduce Golgi Ptdlns4P levels in various cell types, which is mainly mediated by promoting the retention of SACM1L in the Golgi after transfer from the ER. Based on this knowledge and our observation that both glucose and serum deprivation induce ATGL upregulation, we hypothesized that reduction of Golgi Ptdlns4P levels during nutrient shortage might stabilize ATGL and, in turn, promote lipolysis. Firstly, the inventors examined Golgi Ptdlns4P levels via a P4M-EGFP sensor and a Ptdlns4P antibody in HEK293-AAV cell type. In line with previous publications, a reduction of Golgi Ptdlns4P levels by live cell imaging and immunostaining in conjunction with enrichment of SACM1L in the Golgi apparatus after glucose withdrawal from the culture medium was observed (Fig. 3a). These observations were supported by the reduction of total Ptdlns4P levels after glucose deprivation. The glucose deprivation reduced Golgi Ptdlns4P levels independently of the AMPK pathway as activation and suppression of this kinase could not affect Ptdlns4P levels in the Golgi. To study the effects of Golgi Ptdlns4P on lipolysis, the inventors genetically manipulated Golgi Ptdlns4P levels via siRNA-mediated knockdown of PI4KB, the main lipid kinase for Golgi Ptdlns4P generation, as well as SACM1L. Interestingly, it was observed that Golgi Ptdlns4P reduction led to elevated ATGL levels, whereas Golgi Ptdlns4P accumulation decreased ATGL protein abundance in the HEK293T cell type (Fig. 3b). Moreover, the inventors used three *PI4KB* inhibitors to block Golgi Ptdlns4P generation, and all three compounds elevated both endogenous and ectopically expressed ATGL protein levels (Fig. 3c andFig. 8a, b, c). As with glucose deprivation, ATGL protein accumulated in the Golgi when synthesis of Golgi Ptdlns4P was blocked (Fig. 3d, e), indicating that Golgi Ptdlns4P levels are crucial in determining the stability of ATGL in the Golgi. As glucose deprivation promoted ATGL-mediated lipolysis in different cell types, the impact of Golgi Ptdlns4P levels on this process in liver and fat cell types was examined. It was confirmed that the reduction of Golgi Ptdlns4P increased both endogenous and ectopically expressed ATGL levels while upregulation of the former had the opposite effect on ATGL stability in liver cell type (Fig. 3f, g and Fig. 9a). Likewise, genetic depletion and pharmacological inhibition of *PI4KB* promoted ATGL stability and lipolysis levels whereas SACM1L depletion suppressed this process in adipocytes (Fig. 3h-m).

Considering the relationship between glucose deprivation and Golgi Ptdlns4P, as well as their roles in ATGL stabilization, the inventors next studied whether glucose deprivation could regulate ATGL degradation via Golgi Ptdlns4P. The inventors first analyzed ATGL regulation in response to glucose deprivation in the presence of SACM1LK2A overexpression), a Golgi apparatus resident mutant of SACM1L to reduce Golgi Ptdlns4P levels. Furthermore, the inventors also depleted Ptdlns4P in the Golgi apparatus by UCB9608 mediated inhibition of Ptdlns4P formation to examine ATGL response to glucose deprivation. The loss of ATGL regulation after SACM1LK2A overexpression and UCB9608 treatment together suggests that reduction of Golgi Ptdlns4P levels is not only required for glucose deprivation induced ATGL upregulation but also sufficient to increase ATGL levels even under high glucose conditions. Collectively, it could be shown that glucose availability regulates the distribution of SACM1L between ER and Golgi apparatus and thus modulates Golgi Ptdlns4P levels, which in turn governs lipolysis by regulating ATGL stability in the Golgi.

### Example 4: The E3 ligase complex CUL7^{FBXW8} regulates ATGL in the Golgi apparatus

Although ATGL is regulated in the Golgi apparatus, it remains unclear how Golgi Ptdlns4P levels could modulate ATGL stability. Given that proteasome mediated degradation and autophagy are the main pathways that can affect the degradation of intracellular proteins, the inventors blocked both processes by MG132 and Chloroquine treatment, respectively. Interestingly, only MG132 treatment led to the cellular accumulation of ATGL protein, indicating that ATGL degradation is executed by the proteasome. Post-translational modification of substrates via K48-linked polyubiquitylation through E3 ligases acts as the main signal for proteasome-mediated degradation. The inventors thus examined whether lysine ubiquitylation of ATGL is dispensable for its regulation upon glucose deprivation. Therefore, the inventors constructed a lysine null ATGL mutant, and analyzed its accumulation after treatment with glucose-free medium or in response to an addition of 2-DG. In contrast to wild-type ATGL, the lysine null ATGL mutant was unresponsive to either treatment, suggesting that lysine modification is essential. Furthermore, K48-linked polyubiquitylation of ATGL was reduced upon glucose deprivation. These observations suggest the presence of a Golgi resident E3 ligase to sense glucose availability that can polyubiquitylate ATGL for proteasome-mediated degradation.

To identify the E3 ligase responsible for glucose deprivation-induced ATGL accumulation in the Golgi, the inventors performed a small screen of E3 ligases, which are reportedly localized to the Golgi apparatus, and the well-known Golgi Ptdlns4P effectors. Among the tested candidates, it was observed that only depletion of CUL7 and FBXW8, components of the E3 ligase complex CUL7^{FBXW8}, was able to significantly elevate ATGL levels in both the whole-cell extract and the Golgi fraction (Fig. 4a-c). Of note, both CUL7 and FBXW8 were barely detected in the vesicle fraction albeit that ATGL levels were elevated in both Golgi and vesicle fractions. Furthermore, depletion of either component substantially reduced LD levels, which was dependent on the presence of ATGL. The scaffold protein CUL7 interacts with the E3 ligase RBX1, adaptor protein SKP1 and substrate receptor FBXW8 to form a functional E3 ligase complex, formation of which is crucial to stabilize these proteins. Moreover, the presence of a positively charged polybasic region, the putative interactor of negatively charged Ptdlns4P, in the N-terminus of FBXW8 might allow the CUL7^{FBXW8} E3 ligase complex to sense glucose availability by recruitment to the Golgi. This makes this complex a promising candidate to modulate glucose deprivation-induced enhancement of ATGL-driven lipolysis. Prior to testing this hypothesis, the inventors first analyzed the function of CUL7^{FBXW8} in liver and adipocyte cell types. Depletion of *CUL7^{FBXW8}* resulted in higher ATGL protein levels, as well as an ATGL-dependent decline of LD abundance and lipolysis, respectively (Fig. 4d-f). These findings led the inventors to further probe the mechanistic role of CUL7^{FBXW8} in the regulation of ATGL regulation and thus we measured the physical interaction between FBXW8, the substrate receptor of *CUL7^{FBXW8}* E3 ligase complex, and ATGL by co-immunoprecipitation (Co-IP). The inventors observed a direct interaction of FBXW8 and ATGL, as well as reduced K48-linked polyubiquitylation levels of ATGL, after CUL7^{FBXW8} depletion, which demonstrates that CUL7^{FBXW8} functions as the E3 ligase complex and interacts with ATGL in the Golgi for polyubiquitylation and proteasome-mediated degradation. Lastly, the inventors examined ATGL regulation in response to glucose deprivation in the absence of CUL7^{FBXW8} and found that glucose sensing was abolished under these conditions (Fig. 4g). Taken together, these data show that the E3 ligase complex CUL7^{FBXW8} is essential to regulate polyubiquitylation and degradation of ATGL in the Golgi in response to glucose deprivation.

### Example 5: Golgi Ptdlns4P Regulates CUL7^{FBXW8} function

CUL7 and FBXW8 were previously shown to localize to the Golgi of certain cell types. To understand how CUL7^{FBXW8} senses glucose availability and, in turn, regulates ATGL degradation, the inventors next examined the cellular localization of CUL7 and FBXW8 by immunostaining. In line with previous reports and our fractionation result, endogenous CUL7 was highly enriched in the Golgi, instead of vesicle (Fig. 4c). Unexpectedly, however, both endogenously and ectopically expressed FBXW8 exhibit a dispersed pattern, with a certain portion of localization in the Golgi (Fig. 4c). In contrast, the vesicle fraction contains barely detectable levels of endogenous FBXW8 protein (Fig. 4c). Considering the existence of the polybasic region in the N terminus of FBXW8, the inventos hypothesized that Golgi Ptdlns4P might be able to recruit FBXW8 to the Golgi where it would be assembled into a functional E3 ligase complex with CUL7, thus enabling CUL7^{FBXW8} to sense glucose availability and regulate ATGL levels. Therefore, the inventors examined CUL7 and FBXW8 levels in the Golgi upon glucose deprivation via fractionation and immunostaining. As expected, FBXW8 instead of CUL7 displayed reduced levels in the Golgi (Fig. 5a). Next, the inventors depleted Golgi Ptdlns4P and observed reduced FBXW8 distribution to the Golgi (Fig. 5b), indicating that its distribution is dependent on Golgi Ptdlns4P. To demonstrate the interaction between the polybasic region of FBXW8 and Ptdlns4P, the inventors built FLAG-tagged wild-type FBXW8 and an FBXW8 mutant in which all lysine and arginine residues in the polybasic region of FBXW8 were mutated to alanine and stably expressed them in HEK293T cell type. The inventors purified recombinant FBXW8 proteins from stable cell lines and examined their binding capacity with various lipid species in Membrane Lipid Strips. Among these tested lipid species, Ptdlns4P displayed binding capacity to wild-type FBXW8 whereas the interaction was lower between Ptdlns4P and the mutant FBXW8. These data were confirmed using PIP Strips, which also demonstrated a reduced binding capacity of Ptdlns4P to mutant versus wild-type FBXW8 (Fig. 5c). Moreover, the inventors used Ptdlns4P-conjugated agarose beads to precipitate mutant and wild-type FBXW8, and the inventors showed that Ptdlns4P beads also exhibited a stronger interaction with wild-type FBXW8 compared to the mutant.

After demonstrating the physical interaction between the polybasic region of FBXW8 and Ptdlns4P, the inventors next examined whether the recruitment of FBXW8 to the Golgi was affected by glucose availability. In agreement with its Ptdlns4P interaction capacity, wild-type FBXW8 levels in the Golgi apparatus were reduced upon glucose deprivation, while mutant FBXW8 was not sensitive to glucose levels (Fig. 5d, e). In conclusion, glucose deprivation leads to lower levels of Golgi Ptdlns4P. This negatively charged phospholipid interacts with the positively charged polybasic region of FBXW8 and affects the recruitment of FBXW8 to the Golgi apparatus, where the *CUL7^{FBXW8}* E3 ligase complex is assembled to polyubiquitylate ATGL for proteasomal degradation (Fig. 5f).

### Example 6: Golgi Ptdlns4P and CUL7^{FBXW8} regulates ATGL-driven lipolysis in mice

The data identifies Golgi Ptdlns4P as the central player regulating ATGL-driven lipolysis, thus coupling glucose availability and intracellular FA liberation to regulate energy homeostasis. Therefore, the inventors next focused on liver ATGL regulation as a key factor in hepatic lipolysis and TG accumulation, especially under conditions of fatty liver pathogenesis. To manipulate Golgi Ptdlns4P levels in the liver, the inventors administered AAV8-U6-gRNA-TBG-Cre viruses to *LSL-spCAS9* mice with the gRNA pools targeting either *Pi4kb* or *Sacm1l* specifically in hepatocytes. This approach led to an efficient depletion of *PI4KB* protein levels in the liver 2 weeks after AAV8 administration (Fig. 6a). In the absence of PI4KB, ATGL protein levels were significantly upregulated (Fig. 6a). Global depletion of *Sacm1l* has been reported to cause embryonic lethality, and it was found that liver-specific depletion of SACM1L also led to death 2 weeks after AAV8 administration, which impedes any mechanistic investigation and demonstrates the relevance of SACM1L to other pathways. Thus, the inventors next abrogated *Cul7* and *Fbxw8* specifically in hepatocytes using the same strategy as highlighted above. Depletion of CUL7 led to the destabilization of FBXW8 in the liver but not vice versa (Fig. 6b). This is in contrast to previous reports demonstrating that these two factors can stabilize each other. The inventors observed increased ATGL levels after knockout of *Cul7* and *Fbxw8* in the liver (Fig. 6b), indicating a consistent *in vivo* role for CUL7^{FBXW8}. Therefore, we next examined whether the E3 ligase complex CUL7^{FBXW8} is responsible for hepatic ATGL upregulation in response to glucose deprivation. We thus administered 2-DG to wild-type and *Cul7* and *Fbxw8* double knockout mice for 1 hour. This treatment led to an upregulation of ATGL levels in livers of wild-type mice but not in the absence of CUL7^{FBXW8} (Fig. 6c), underscoring the essential role of CUL7^{FBXW8} in regulating glucose deprivation induced ATGL stabilization.

Upregulation of hepatic ATGL levels is reported to be highly effective against TG deposition in the fatty liver. Therefore, the inventors examined the physiological and pathophysiological effects in non-alcoholic fatty liver disease (NAFLD) models after genetic and pharmacological manipulation of the Golgi PtdIns4P-CUL7^{FBXW8}-ATGL axis. The inventors quantified TG levels in livers after induction of hepatocyte specific *Pi4kb* knockout under a normal chow diet (NCD) feeding condition. Indeed, PI4KB depletion decreased hepatic TG levels (Fig. 6d). Moreover, when PI4KB was ablated in a mouse model of hepatic steatosis induced in a 12-week high-fat diet (HFD) model, TG deposition in the fatty liver was significantly reduced 8 weeks after virus administration (Fig. 6d). Similarly, the inventors determined hepatic TG levels in the *Cul7* and *Fbxw8* knockout mice under NCD feeding conditions and found that only CUL7 ablation reduced hepatic TG levels (Fig. 6 e). In contrast, depletion of either factor was able to reduce hepatic TG accumulation in mice challenged with a HFD for 12 weeks (Fig. 6 e). In addition to genetic manipulation, the inventors examined the effect of the PI4KB inhibitor on lipolysis regulation and NAFLD progression. Therefore, wild-type mice were fed a HFD for 12 weeks to induce hepatic steatosis, and subsequently treated with UCB9608, a PI4KB inhibitor reported to be effective in the mouse model with organ engraftment(Fig. 6f). Interestingly, PI4KB inhibition significantly increased plasma FFA levels (Fig. 6g), suggesting a consistent *in vivo* role of Golgi Ptdlns4P in regulating ATGL-driven lipolysis in the adipose tissue. Thus, the inventors compared the ATGL protein levels in different adipose depots and livers from DMSO- and UCB9608-treated mice, and the inventors found the latter displayed significantly greater ATGL protein levels in the iBAT, gWAT and liver with a similar trend in the iWAT. In agreement with the reported role of ATGL upregulation in the adipose tissue, UCB9608 treatment led to higher energy expenditure, possibly due to elevated FAs levels for energy combustion. The enhancement of ATGL-driven lipolysis in the adipose tissue might contribute to increased FA liberation and release, whereas elevation of hepatic ATGL levels could drive intrahepatic lipolysis and reduce hepatic TG levels. Therefore, the inventors determined hepatic TG levels in the livers of UCB9608-treated mice and observed substantially attenuated hepatic steatosis, which requires the presence of ATGL in the liver (Fig. 6h). In conclusion, it was demonstrated that a Golgi PtdIns4P-CUL7^{FBXW8}-ATGL axis regulates hepatic ATGL levels and LD clearance, which could provide a potential target to ameliorate hepatic steatosis.

### Example 7: Reduction of Golgi Ptdlns4P attenuates NASH progression

As the inventors have demonstrated the potential applicability of the identified pathway for treatment of NAFLD, they wanted to investigate further whether targeting Golgi Ptdlns4P production could attenuate the progression of NASH from simple hepatic steatosis. NASH progression is characterized by increased inflammation, as well as fibrosis, and currently lacks an approved pharmacological treatment (Loomba, R. et al Cell 184, (2021)). To model NASH in the mouse liver, wild-type mice were housed under thermoneutral conditions on a HFD diet coupled with high glucose- and fructose-containing drinking water for 24 weeks and were afterwards treated with UCB9608 for an additional 12 weeks (Fig. 7a). Interestingly, it was observed that UCB9608 treatment induced a significant loss of body weight and fat mass, in agreement with the enhanced lipolysis and energy expenditure induced by UCB9608 (Fig. 7b-d), implicating the effective role of UCB9608 in metabolic improvement. Hence, the inventors further examined TG levels and ALT and AST activity in the plasma from NASH mice. The reduction of these parameters upon UCB9608 treatment indicates alleviated hepatic steatosis and injury, leading the inventors to perform pathohistological analyses of the livers from the treated NASH mice compared to those from vehicle-treated control NASH mice (Fig. 7e, f). The inventors first measured the liver mass and hepatic TG and fatty acid oxidation levels prior to histological analyses and observed that UCB9608 treatment reduced liver mass/body weight ratio and hepatic TG levels, in conjunction with significant elevation of fatty acid oxidation (Fig. 7g, h). Next, the inventors conducted histological analyses including Hematoxylin and Eosin staining (H&E), Oil Red O staining (ORO), cleaved caspase-3 and Gomori Green Trichrome staining to determine hepatic steatosis, inflammation and injury, as well as fibrosis grade. Interestingly, steatosis grade, liver injury, inflammation levels, fibrosis grade and NAFLD activity score were significantly lower in the livers from UCB9608-treated mice, suggesting suppression of NASH progression via UCB9608 treatment, albeit the NASH induction in these murine livers were relatively mild compared to clinical NASH biopsies from human patients (Fig. 7i). The mitigation of inflammation levels upon UCB9608 treatment was further corroborated by quantifying transcript levels of inflammatory markers (Fig. 7j). Similarly, hepatic fibrosis, a very important pathophysiological index that defines NASH, was also analyzed by quantifying transcript levels of fibrosis markers. In agreement, UCB9608 treatment also substantially ameliorated the degree of fibrosis-related marker expression in the livers of the treated NASH mice compared to the vehicle-treated control NASH mice (Fig. 7k).

The progression of NASH may lead to cirrhosis and possibly hepatocellular carcinoma, and both pathological situations require a liver transplantation to circumvent liver failure. However, liver grafts cannot be transplanted when they are steatotic, thus requiring potential strategies to defatten steatotic livers for clinical use. Given the ability of UCB9608 to ameliorate of hepatic TG levels in the mouse models, the inventors tested the defattening effect of UCB9608 on a steatotic human liver during ex *vivo* via a perfusion system (Clavien, P.A. et al. Nat Biotechnol 40, (2022)). The inventors obtained a discarded human liver graft with 15% macrovesicular steatosis and perfused the liver with blood for 8 days. UCB9608 was administered daily to reach a target concentration of 10 µM (Fig. 7I). The graft displayed normal bile acid excretion and oxygen consumption, suggesting healthy status of the graft during the perfusion. Interestingly, the inventors detected non-esterified fatty acids (NEFAs) from dialysate during perfusion, suggesting an induction of lipolysis that resulted in the release of FAs. To study the time course of liver defattening, the inventors sampled biopsies from both lobes to determine ATGL and TG levels. Consistent with the findings above in the mice, the inventors observed substantially elevated ATGL levels after UCB9608 treatment in conjunction with reduced TG deposition while ATGL and hepatic TG levels remained unchanged in the two control grafts, indicating a defattening effect mediated by ATGL-driven lipolysis upon UCB9608 administration (Fig. 7 m, n). Taken together, the discovery of Golgi Ptdlns4P-mediated ATGL stabilization in response to glucose deprivation potentiates a new therapeutic strategy to counteract NASH progression via blocking Golgi Ptdlns4P generation and also serves as a potential therapeutic approach to repair steatotic liver grafts ex *vivo.*

### Conclusion

It is well known that the endocrine system plays important roles in glucose and FA handling during different nutritional states. Elevation of plasma glucose triggers insulin secretion from β cell type to promote glucose uptake in multiple tissues, including the adipose tissue and liver. Insulin can also promote *de novo* lipogenesis to produce FAs using excess glucose as a carbon source, but at the same time it suppresses lipolysis to reduce FA release from lipid stores. Conversely, glucagon secreted from α cell type upon low plasma glucose conditions, or norepinephrine released from the sympathetic nervous system in response to starvation or cold exposure, increases lipolysis through ATGL and HSL phosphorylation in hepatocytes and adipocytes. Typically, such extracellular hormones rapidly initiate systemic response to acute physiological alterations. However, hormone signaling usually does not act chronically due to corresponding receptor desensitization. This process is exemplified by classical β-arrestin-mediated desensitization of β1 and β2 adrenergic receptors and non-canonical desensitization of β3 adrenergic receptor via striking transcriptional downregulation of receptor gene expression upon ligand binding, which might explain the inventor's results that elevated pHSL levels were reversed in the adipose tissue upon long-term glucose deprivation. This homeostatic feedback loop likely necessitates cells to develop intrinsic mechanisms to tune lipolysis to nutrient supplies as a complementary process to extracellular hormone-mediated regulation of lipid metabolism so that the organism can better meet complex physiological demands. Indeed, here the inventors have identified a novel intrinsic mechanism involving the central role of Golgi Ptdlns4P, which senses glucose levels and interacts with E3 ligase complex CUL7^{FBXW8}. This intrinsic mechanism might reshape our understanding regarding the exact contribution of insulin signaling to the lipolysis regulation as insulin could also modulate intracellular glucose levels to initiate the Golgi PtdIns4P-CUL7^{FBXW8}-ATGL axis for lipolysis regulation. Reduction of HSL phosphorylation is believed to be the main mediator of the anti-lipolytic role of insulin. Here the inventors found that insulin does not suppress pHSL under conditions of glucose deprivation, suggesting that glucose itself is an important mediator of lipolytic function and possibly mediates the anti-lipolytic effect of insulin. This insulin signaling-independent mechanism might be especially relevant in insulin resistance as we found that patients with type 2 diabetes display increased ATGL levels upon fasting. Nevertheless, insulin receptor substrate 1 (IRS1), a critical mediator of insulin signaling, is reported to be a substrate of CUL7^{FBXW8}, suggesting a crosstalk between insulin and Golgi Ptdlns4P-mediated regulation of lipolysis.

As the key mediator of Golgi Ptdlns4P to regulate ATGL-driven lipolysis, FBXW8 is recruited to the Golgi for the assembly of E3 ligase complex CUL7^{FBXW8}. Previous reports have shown that *CUL7^{FBXW8}* assembly promotes the stability of CUL7 and FBXW8 as loss of either factor strongly reduces the protein levels of the other. Indeed, the inventors found depletion of CUL7 leads to destabilization of FBXW8 in all the cell types used in the present work, including the liver. As a consequence, FBXW8 levels in both Golgi and WCE are reduced since FBXW8 is less stable in the cytosol without being assembled into CUL7 scaffold. In contrast, the inventors found that depletion of FBXW8 could only destabilize CUL7 in iBAs, highlighting the more important role of the scaffold protein CUL7 in stabilizing the E3 ligase complex. Functionally, CUL7 also has a stronger effect on ATGL-driven lipolysis as CUL7 depletion in HepG2 and HEK293AAV cell types *in vitro* and in the liver *in vivo* leads to lower LD abundance or TG levels compared to FBXW8 ablation.

Adipose tissue stores massive levels of FAs in LDs and is the source of a substantial supply of intracellular and extracellular FAs via lipolysis to meet the energy demands of its own cells and other tissues. In contrast to adipocytes, most non-adipocytes possess fewer LDs, and thus upon glucose or serum deprivation they have to initiate autophagic degradation of non-essential cellular parts to provide carbon sources to generate LDs in proximity to mitochondria so that any intracellular lipolysis that takes place allows for proximal FAs flux to fuel fatty acid oxidation. The inventors found that ATGL is stabilized in the Golgi apparatus of the starved cells. This response likely primes lipolysis activation in the starved cells for ATGL translocation from the Golgi apparatus to the LDs once the latter are generated. These mechanisms ensure timely and efficient mobilization of less important cell components to overcome nutritional shortages in non-adipocytes, in addition to the FAs taken up from the circulation. In addition, abundant LDs can be artificially induced by oleic acid-containing medium in non-adipocytes. Under this condition, glucose or serum starvation can induce autophagic degradation of the LD coat proteins perilipin 2 and 3 (PLIN2/3), the clearance of which can empty the LD surface for ATGL localization and action. However, the inventors observed that PLIN1, the predominant LD coat protein in adipocytes, displays no difference in protein levels from various adipose depots after fasting, suggesting that the regulation of lipolysis in adipocytes and non-adipocytes display distinctive features despite existence of a common pathway. Further, lipophagy can directly mobilize FAs from LDs via LAL1. In hepatocytes, lipolysis is responsible for the liberation of FAs from large LDs and subsequently FAs from the resultant smaller LDs are then mobilized by lipophagy.

NASH typically progresses from simple hepatic steatosis and is marked by elevated hepatic lipids, inflammation and fibrosis. Hepatosteatosis is mainly due to elevated fatty acids delivered from the circulation, in addition to elevated hepatic *de novo* lipogenesis, decreased hepatic lipolysis and fatty acid oxidation in the hepatocytes. Inflammation is induced by secretion of inflammatory factors and inflammasome activation in the Kupffer cell type while hepatic stellate cell type are responsible for secretion of collagen that contributes to fibrosis, which is believed to be exacerbated by the inflammation. Based on the inventor's findings and other reported studies, Golgi Ptdlns4P might serve as a potent target to block multiples factors contributing to NASH progression. The inventors show that reducing Golgi Ptdlns4P stabilizes ATGL and promotes intrahepatic lipolysis and LD clearance. Golgi Ptdlns4P also plays pivotal roles in NLRP3 activation via direct recruitment to the Golgi apparatus and thus the inflammation in the NASH liver probably can be directly alleviated by lowering the Ptdlns4P levels. Further, procollagen, after being synthesized in the ER, traffics to the Golgi apparatus whereby it is processed and secreted into the extracellular space via large transport vesicles. The formation of these vesicles might require the contribution of Golgi Ptdlns4P to induce membrane curvature, like certain type of Golgi-derived vesicles. If so, targeting PI4KB is a very promising strategy to directly attenuate steatosis, inflammation and fibrosis in NASH as the inventors observed in their mouse model. More importantly, UCB9608 treatment reduced plasma TG levels in the mouse NASH model, unlike elevated TG secretion and hypertriglyceridemia observed upon administration of an ACC inhibitor in mice and humans.

Additionally, the inventor's study suggested that UCB9608 is able to defatten a steatotic liver during ex *vivo* perfusion, which could allow more grafts to be utilized for transplantation after testing more grafts perfused with UCB9608 as currently steatotic livers are ineligible for such use in the clinic. Interestingly, UCB9608 is also known to exhibit immunosuppressive properties. Considering immunosuppressive agents are necessary to prevent allograft rejection after organ transplantation, it will be a promising option to continue UCB9608 administration after engraftment if it is approved for clinical application.

In summary, the inventor's findings identify a new intrinsic pathway that links glucose availability to lipid availability via glucose sensing by a Golgi Ptdlns4P-ATGL-lipolysis pathway that leads to the upregulation of lipolysis and the increased release of FAs from LD stores upon glucose depletion. By this means cells can better cope with nutrient flux than relying solely on paracrine hormone-mediated regulation of nutrient availability.

### Materials and Methods

### EHC Study

The study was approved by the by the Local Ethics Committee (Bratislava, Slovakia) and conforms to the ethical guidelines of the 2000 Helsinki declaration. All study participants provided witnessed written informed consent before entering the study. EHC was used to determine the *in vivo* insulin sensitivity (M value). In brief, human insulin (Actrapid 100 IU/mL, Novo Nordisk, Bagsvaerd, Denmark) was infused into the antecubital vein in a primed continuous fashion at the dose of 1 mU/kg/min. Blood glucose was measured in the samples from the contralateral antecubital vein in 5 minutes intervals and maintained at euglycemia (5.0 ± 0.25 mM) using variable infusion rate of 20% glucose. The whole-body insulin sensitivity (M value) was calculated from the steady state plasma glucose infusion rate required to maintain euglycemia and expressed per kg body weight per minute, normalized to the steady state insulinemia. Subcutaneous AT samples were taken by needle biopsy from abdominal region in the basal fasted state (prior to administration of insulin and glucose) as well as during the steady state of EHC. Samples were immediately rinsed, blotted and frozen in liquid nitrogen and stored at -80°C. Twenty-four middle-aged sedentary men (age 35.5 ± 1.5 years, BMI 28.9 ± 1.1 kg.m⁻², adiposity 25.7 ± 1.6%) were assigned to insulin sensitive (n=16, M value 0.14 ± 0.02 mg/kg BW/min/insulin µU/ml) and insulin resistant (n=8, M value 0.04 ± 0.01 mg/kg BW/min/insulin µU/ml) subgroups, according to their insulin sensitivity index (M value).

### Human Liver Perfusion

A human liver graft was perfused for 8 days, after being discarded from all Swiss transplant centers. Use of discarded grafts was approved by local authorities (Kantonale Ethik Kommission Zürich KEK Nr. 2017-000412). The graft had an initial weight of 2740g (with gall bladder) before perfusion and was diagnosed with 15% macrovesicular steatosis by the pathological department of the University Hospital Zürich. The liver was procured with a total ischemia time of 40 min after circulatory death of the donor. It was flushed with IGL-1 solution and perfused for 2 h with Belzer MPS at 8°C. The infrahepatic vena cava was closed with a suture and the hepatic artery, portal vein, suprahepatic vena cava and common bile duct were cannulated with steel cannulas. The graft was subsequently perfused with whole blood and a custom built normothermic perfusion machine (Eshmuminov,D. et al, Nat Biotechnol 38, (2020)). Hepatic arterial pressure was controlled to be 80/50 mmHg, allowing for an average flow of 0.25 L/min. A vasodilator, epoprostenol, was automatically infused to keep arterial flow stable. Oxygenation was automatically controlled with a setpoint partial pressure of oxygen equal to 12 kPa. Portal venous flow was controlled to be at 1 L/min, resulting in a pressure of 2 mmHg on average. Further, oxygenation of portal blood was automatically regulated to achieve 65% saturation in the vena cava. Use of an electric pinch valve further allowed for pressure regulation in the vena cava, keeping a constant pressure of 0 mmHg. Glucose levels were automatically controlled by using a real-time glucose sensor (CCIT) and automated insulin infusions, keeping glucose levels at 7 mmol/L from the second day of perfusion onwards. A dialysis bypass was used to clear waste metabolites and control haematocrit levels by using a fixed dialysate influx rate of 200 mL/h and an automatically controlled outflux. Liver function was well preserved for at least 6 days and cell viability, as monitored by ATP analysis and histology, remained until the end of perfusion. After 8 days of perfusion, the liver weight decreased to 1507g (without gallbladder) and was sent for pathological examination. The liver graft finally displays less than 5% macrovesicular steatosis determined by the pathological department of the University Hospital Zürich.

### Human Liver Perfusion - Medication and Perfusate Composition

Perfusate was mixed with 6 units of red blood cell concentrate (A+), 5 units of fresh frozen blood cells, one unit of thrombocytes and 200 mL of 20% human albumin (CSL Behring). An initial bolus of 2.25 g Tazobactam and 500 mg Methylprednisolone was administered after priming. During the perfusion, 1000 U of heparin were infused per hour. Further, 2.25 g of Tazobactam, 3.36 g of ursodesoxycholic acid and 500 mg of methylprednisolone were continuously infused per day. UCB9608 was administered as a daily bolus by adding 300 µL of 50 mM solution directly to the blood reservoir, targeting a final concentration in the perfusate of 10 µM. On the first day, a larger bolus of 600 µL was given.

### Mouse Experiments

All animal experiments were permitted by the Veterinary office of the Canton of Zurich. Both female and male mice were used in the experiments, and they were housed 2-5 littermates per cage in ventilated cages at standard housing conditions (22°C, 12 h reversed light/dark cycle, dark phase starting at 7 am) with normal chow diet (18% proteins, 4.5% fibers, 4.5% fat, 6.3% ashes, Provimi Kliba SA) and water. Health status of all the mouse lines were monitored regularly based on FELASA guidelines. 8-10 weeks old C57BL/6N male mice were fasted for 24 h or injected with 2-DG (2 g/kg BW) to induce glucose deprivation for different durations. Female *ROSA26-LSL-spCas9* mice (The Jackson Lab strain) were intravenously injected with AAV8-U6-Pi4kb gRNAs-TBG-Cre, AAV8-U6-*Sacm1l* gRNAs-TBG-Cre, AAV8-U6-Cu/7 gRNAs-TBG-Cre and AAV8-U6-Fbxw8 gRNAs-TBG-Cre virus pools to express six gRNAs targeting individual gene and CAS9 in the liver at a dose of 3×10¹¹ genome copies/mouse. Two weeks later, livers were harvested for protein and RNA extraction after 6 hours fasting. To determine liver TG levels under NCD feeding condition, 4 weeks after AAV administration livers were harvested after 6 hours fasting. Also 4 weeks after AAV administration, the liver specific Cul7&Fbxw8 double knockout mice were injected with 2-DG (2 g/kg BW) to induce 1 hour glucose deprivation prior to liver harvesting for protein extraction.

Male mice were used for NAFLD induction via feeding on HFD. *ROSA26-LSL-spCas9* mice were injected with AAV-U6-Cu/7 gRNAs-TBG-Cre and AAV-U6-Fbxw8 gRNAs-TBG-Cre virus pools at a dose of 3×10¹¹ genome copies/mouse and fed in HFD for 12 weeks and livers were harvested after 6 hours fasting. To study PI4KB in NAFLD progression, male *ROSA26-LSL-spCas9* mice were fed in HFD for 12 weeks to induce fatty liver before induction of hepatocyte specific depletion of PI4KB via intravenous administration of AAV8. After another 8 weeks, mice were dissected to harvest liver after 6 hours fasting. Likewise, male C57BL/6N wild-type mice and liver specific *Atgl* knockout mice (*AtgILKO*) were challenged with HFD for 12 weeks to induce fatty liver prior to administration of UCB9608 mixed in HFD (5 mg/kg) for another 8 weeks. Then the mice were dissected to harvest tissues and sample blood after 6 hours fasting
*Atgl* floxed mice (The Jackson Lab strain) were crossed with *Albumin-Cre* (The Jackson Lab strain) and *Adip-CreERT2* strains to obtain *Alb-Atgfl*^{*fl*/*fl*} and *AdipcreERT*2*-Atgl*^{*fl*/*fl*} mice (Sitnick,M.T. et al, Diabetes 62, (2013)), which deplete ATGL protein in the liver and the adipose depots (induced after tamoxifen gavage), respectively. Male mice from both strains were injected with 2-DG (2 g/kg BW) to induce glucose deprivation for 6 hours prior to tissue and blood harvesting.

### 2-DG and insulin administration

Wild type C57BL/6N mice were administered with saline or 2-DG (2 g/kg BW) via intraperitoneal injection for 5.5 hours treatment, followed by insulin treatment (Actrapid, human insulin at dose of 0.75 U/kg BW) for 30 minutes. The mice were then euthanized to harvest gonadal white adipose tissue and blood to study the acute anti-lipolytic effect of insulin under the background of glucose deprivation. To study the long term anti-lipolytic effect of insulin during glucose deprivation, wild type C57BL/6N were co-administered with 2-DG (2 g/kg BW) and insulin (0.75 U/kg BW) for 6 hours treatment. Afterwards, mice were euthanized for blood sampling.

### Administration of UCB9608 in murine NASH model

To model NASH in murine liver, male C57BL/6N mice were challenged with HFD for 24 weeks in conjunction with 18.9 g/L glucose&23.1 g/L fructose containing drinking water under thermoneutral housing condition (29°C) for 12 weeks before the administration of UCB9608 through HFD (15 mg/kg) for another 12 weeks. Then the mice were analysed in EcoMRI for body composition measurement before 6 hours fasting, followed by dissection to harvest liver and sample blood.

### Indirect Calorimetry

8-10 weeks old C57BL/6N male mice were fed on HFD mixing with DMSO or UCB9608 (5 mg/kg) for 3 weeks before indirect calorimetry measurements were performed with Phenomaster (TSE systems) according to the manufacturer's instructions. Animals were single caged and adapted to the metabolic cage for 48h prior to metabolic recording. The energy expenditure between both groups was analyzed via ANCOVA method and RER was analyzed via two-way ANOVA method.

### Cell Culture

Immortalized murine brown preadipocytes (iBAs) were kindly provided by Prof. Klein lab (Klein,J. et al. BioEssays 24, (2002)). IBAs were cultured on collagen-coated plates in DMEM medium (high glucose) with 10% FBS plus 1% Pen/Strep. Once preadipocytes reached confluence, adipogenesis was induced by differentiation medium containing IBMX (500 µM), dexamethansone (1 µM), insulin (20 nM), T3 (1 nM) and indomethacin (125 µM) for 2 days. Afterwards, iBAs were refreshed with maintenance medium containing insulin (20 nM), T3 (1 nM). At day 5, differentiated iBAs were re-seeded to reduce cell density. All the experiments were routinely performed at day 9. For siRNA mediated knockdown experiments, Lipofectamine RNAiMAX reagent mediated siRNAs (100 nM) delivery was performed to knock down target genes for 72 h in iBAs after one day recovery from replating. To deprive glucose in the iBAs culture, we either added 2-DG at indicated concentrations to the culture medium or refresh cells with DMEM medium containing different glucose concentrations prepared from the DMEM medium (Gibco, A1443001) with same concentration of insulin and T3 as the maintenance medium.

HEK293T and HEK293-AAV cell types were cultured in DMEM (high glucose) with 10% FBS and 1% Pen/Strep. HEK293-AAV cell type was used for imaging whereas HEK293T cells were adopted for the rest of the work. To overexpress EGFP-P4M, EGFP-SACM1L, EGFP-SACM1LK2A, ATGL-FLAG, ATGL-HA, ATGLK0-HA and FBXW8-FLAG in the HEK cells, Lipofectamine 2000 was used to deliver these plasmids for 24 h expression at 60-80% cell confluence according to the manufacturer's instructions, followed by specific treatments based on experimental designs. To image LDs abundance, HEK293-AAV cell type was induced by 200 µM oleic acid (OA, 01008 from Sigma) conjugated to bovine serum albumin (BSA, A6003 from Sigma) to generate LDs for 24 h prior to 4% PFA fixation. Afterwards, cells were stained by BODIPY 493/503 and Hoechst 33342 for LDs and nuclei labelling, respectively. In other conditions, HEK cells were not treated with OA containing medium. To deprive glucose in the HEK cells, we either added 2-DG at indicated concentrations to the culture medium or refresh cells with 4.5 g/L and 0 g/L glucose containing DMEM medium prepared from the same DMEM medium (Gibco, A1443001). To deprive amino acids or fatty acids, we used amino acids free medium (FUJIFILM, 048-33575) and added 5% fatty acids free BSA (A6003, Sigma) to the normal medium, respectively.

HepG2 cell type was cultured in RPMI medium with 10% FBS and 1% Pen/Strep on collagen-coated plates. Lipofectamine RNAiMAX reagent mediated siRNA (100 nM) was delivered to cells at the suspended state for 48 h. Different from HEK cells, it is not necessary to induce LDs via OA treatment in that there exist LDs in the HepG2 cell type at normal culture medium. The inventors treated HepG2 cells with 10 µM PF04620110 (DGAT1 inhibitor, Sigma) & PF-06424439 (DGAT2 inhibitor, Sigma) for 48 hours or with 5 µg/mL BFA (B5936, Sigma) for 6 hours to deplete LDs and intact Golgi in the HepG2 cell type respectively before glucose deprivation. To deprive glucose in the HepG2 cell type, we either added 2-DG at indicated concentrations to the culture medium or refresh cells with RPMI medium containing different glucose concentrations prepared from the same RPMI medium (Gibco, 11879020).

All the cell lines used in this work were regularly tested negative for mycoplasma contamination. All the siRNAs used in these cell lines were listed in Table 1.

**Table 1: SiRNA sequences used in this study.**

| Gene symbol | Species | Sense siRNA sequence (with dTdT overhanq) | SEQ ID NO |
|---|---|---|---|
| *Pi4kb* | Mouse | #1 CCAAGUUACGGAAGCUAAU | 1 |
| | | #2 GCCCAAUUAUGACAAUGAU | 2 |
| *Sacm1l* | Mouse | #1 GCAUCUGAAGCUGCAUAUU | 3 |
| | | #2GGUGGCAGGUAAUUAUCUU | 4 |
| *Cul7* | Mouse | #1 GGAAGAAGCCAUUGGAGAA | 5 |
| *Fbxw8* | Mouse | #1 GAUCCAUGCCUAUGAAUUU | 6 |
| *PI4KB* | Human | #1 GGGAUGACCUUCGGCAAGA | 7 |
| *SACM1L* | Human | #1 GCCUAUUAUCAUGGUUGUU | 8 |
| *CUL7* | Human | #1 GCACAAGAGCGAGAAGGAA | 9 |
| | | #2 UGAGAUCCUAGCUGAACUG | 10 |
| *FBXW8* | Human | #1 GGGAUUAUCGGAUGAACCA | 11 |
| | | #2 GGGAUUUAAGGACCGGAAA | 12 |
| *HACE1* | Human | #1 UAUAGCGCUGAUGUCAACA | 13 |
| *STU81* | Human | #1 CGCUGGUGGCCGUGUAUUA | 14 |
| *SH3RF1* | Human | #1 CAGAGGCCUUGGAAACCUG | 15 |
| *KLHL20* | Human | #1 GCCUGCUGUGAAUUCUUAA | 16 |
| *PJA2* | Human | #1 GAGAUGAGUUUGAAGAGUU | 17 |
| *CUL3* | Human | #1 GAGAAGAUGUACUAAAUUC | 18 |
| *CUL5* | Human | #1 GACACGACGUCUUAUAUUA | 19 |
| *ASB13* | Human | #1 GGAGAAGAUUGCCAAGUUA | 20 |
| *FBXL 14* | Human | #1 GCUACAACCUCACCGACAA | 21 |
| *FBXW4* | Human | #1 GGAUGCAGCUAGAGGAUGA | 22 |
| *HERC1* | Human | #1 CGGCAUGGAUGAACAAAUU | 23 |
| *KBTBD8* | Human | #1 GAGCAUGAACAGAAUGAAA | 24 |
| *UBAC* | Human | #1 AGAGAUGAGCUGACGGAAA | 25 |
| *RNF121* | Human | #1 CCUUCUCAUCUCUGGCCGA | 26 |
| *HECW1* | Human | #1 CAGCUGCAAUUCCGAUUUG | 27 |
| *HECVV2* | Human | #1 GUGGGUACCUCCAGUUUAA | 28 |
| *NEDD4* | Human | #1 GGCGAUUUGUAAACCGAAU | 29 |
| *NEDD4L* | Human | #1 GAAUAUCGCUGGAGACUCU | 30 |
| | | #2 GAGUCCUAUCGGAGAAUUA | 31 |
| *SMURF1* | Human | #1 GGGCUCUUCCAGUAUUCUA | 32 |
| *SMURF2* | Human | #1 GAUGAGAACACUCCAAUUA | 33 |
| *ITCH* | Human | #1 GCUGUUGUUUGCCAUAGAA | 34 |
| COP1 | Human | #1 GCUGUGGUCUACCAAUCUA | 35 |
| *CBLC* | Human | #1 GGGAAAGUACUGUGGACAC | 36 |
| *HECTD3* | Human | #1 GCGGGAACUAGGGUUGAAU | 37 |
| *OSBP1* | Human | #1 CACAACUGUACACAACAUU | 38 |
| *CRET* | Human | #1 GAAGAUGACUUUCCUACAA | 39 |
| *ORP1* | Human | #1 GCCAGUGCCGGAUUCUGAA | 40 |
| *ORP5* | Human | #1 UGGGUGGGAAGGUCACCAU | 41 |
| *ORP9* | Human | #1 GAGGAGCACAAGAGCGUUA | 42 |
| *ORP10* | Human | #1 CAACCCAACCAACACCAUU | 43 |
| *ORP11* | Human | #1 GAGACUUAAUUAGACGAAU | 44 |
| *VAPA* | Human | #1 GGCAAAACCUGAUGAAUUA | 45 |
| *VAPB* | Human | #1 GUAAGAGGCUGCAAGGUGA | 46 |
| *FAPP1* | Human | #1 CGAAGAACCUACUCAGAUA | 47 |
| *FAPP2* | Human | #1 GAGAUAGACUGCAGCAUAU | 48 |
| *GOLPH3* | Human | #1 GUUAAGAAAUGUACGGGAA | 49 |
| *GP73* | Human | #1 GCAUCAUCGUCUUGGGCUU | 50 |
| | | #2 GCAGGAUGUCCUCCAGUUU | 51 |
| | | #3 GCCAGUGCAUCAAUCAGAU | 52 |
| | | #4 GGAUUCAAAGAGACAAGUU | 53 |
| *ATGL* | Human | #1 GCGAGAAGACGUGGAACAU | 54 |
| | | #2 CCUGCCACUCUAUGAGCUU | 55 |
| | | #3 GCACCUGUGCCUUAAUCUU | 56 |

### RNA extraction and quantitative PCR

Total RNA was extracted from adipose depots, liver or *in vitro* cell cultures via Trizol reagent (Invitrogen) according to the manufacturer's instructions. Genomic DNA was removed from the crude RNA samples by DNase treatment (NEB BioLabs). For individual sample, 1 µg of total RNA was used to convert into cDNA library by using the High-Capacity cDNA Reverse transcription kit (Applied Biosystems, 4368813). Quantitative PCR was performed using KAPA SYBR FAST Universal kit (Roche, KK4618) on ViiA7 (Applied Biosystems) and the relative transcript levels were normalized to 3684 expression via ΔΔCt method. Primer sequences were listed in Table 2.

**Table 2: Sequences of qPCR primers used in this study.**

| Gene symbol | Species | Primer set for qPCR | SEQ ID NO |
|---|---|---|---|
| *Emr1* | Mouse | F: CAGCTGTAACCGGATGGCAAACTT | 57 |
| | | R: TGAACAGCACGACACAGCAGGAA | 58 |
| *Ccl2* | Mouse | F: CATCCACGTGTTGGCTCA | 59 |
| | | R: GATCATCTTGCTGGTGAATGAGT | 60 |
| *Tnf α* | Mouse | F: CAGGGGCCACCACGCTCTTC | 61 |
| | | R: TACGACGTGGGCTACAGGCTTGTC | 62 |
| *II1b* | Mouse | F: AGTTGACGGACCCCAAAAG | 63 |
| | | R: AGCTGGATGCTCTCATCAGG | 64 |
| *Tgfb1* | Mouse | F: GCTGAACCAAGGAGACGGAA | 65 |
| | | R: ATGTCATGGATGGTGCCCAG | 66 |
| *Col1a1* | Mouse | F: CATGTTCAGCTTTGTGGACCT | 67 |
| | | R: GCAGCTGACTTCAGGGATGT | 68 |
| *Col1a2* | Mouse | F: GCAGGTTCACCTACTCTGTCCT | 69 |
| | | R: CTTGCCCCATTCATTTGTCT | 70 |
| *Timp1* | Mouse | F: GATATGCCCACAAGTCCCAGAACC | 71 |
| | | R: GCACACCCCACAGCCAGCACTAT | 72 |
| *Acta2* | Mouse | F: ACTGGGACGACATGGAAAAG | 73 |
| | | R: GTTCAGTGGTGCCTCTGTCA | 74 |
| *Atgl* | Mouse | F: tgaccatctgccttccaga | 75 |
| | | R: tgtaggtggcqcaaqaca | 76 |
| *36B4* | Mouse | F: gccgtgatgcccagggaaga | 77 |
| | | R: catctgcttggaqcccacqtt | 78 |
| *ATGL* | Human | F: ctccaccaacatccacgag | 79 |
| | | R: ccctgcttgcacatctctc | 80 |
| *36B4* | Human | F: ccaggcgtcctcgtggaagt | 81 |
| | | R: tgctgcatctgcttggagccca | 82 |

### Molecular cloning

Human ATGL-FLAG and ATGLK0-FLAG expression constructs were generated as described previously (Ding, L. et al. Nat Metab 1-14 (2021)). Based on these vectors, the inventors designed primers to replace FLAG with HA tag and insert them into pEGFP-N1-FLAG vector (Addgene, #60360) with stop codon before FLAG-EGFP CDS to obtain ATGL-HA and ATGLK0-HA expression constructs. Human *SACM1L* and SACM1LK2A CDS were cloned from vector mCherry-FKBP-SAC1 (Addgene, #108127) and inserted into pEGFP-C1-FLAG vector (Addgene, #46956) to have EGFP tag in the N-terminus. Human FBXW8-FLAG CDS was cloned from HEK293T cDNA library and inserted into pLenti-CMV-MCS-BSD vector to obtain pLenti-FBXW8-FLAG construct. Human FBXW8 mutant CDS with substitution of lysines and arginines into alanines in the N terminus was ordered from Vectorbuilder Inc. and the CDS was then inserted into pLenti-CMV-MCS-BSD vector to obtain pLenti-FBXW8 mutant-FLAG construct. Scarlet-Giantin CDS was cloned from plasmid pmScarlet-Giantin-C1 (Addgene #85048) and inserted into pLenti-CMV-MCS-BSD vector to obtain pLenti-Scarlet-Giantin construct. Sequences to transcribe six different gRNAs targeting individual gene were ordered from Microsynth AG and inserted into AAV-U6-TBG-Cre vectors so that AAV-U6-Pi4kb gRNA-TBG-Cre, AAV-U6-Sacm1l gRNA-TBG-Cre, AAV-U6-Cul7 gRNA-TBG-Cre and AAV-U6-Fbxw8 gRNA-TBG-Cre were constructed. All the plasmids for cell culture work were extracted by NucleoBond Xtra-Midi kit (Macherey-Nagel, 740410.100).

### Virus packaging and stable cell line construction

For lentivirus packaging, the inventors used 3rd generation system. The pLenti-Scarlet-Giantin, pLenti-FBXW8-FLAG or pLenti-FBXW8 mutant-FLAG plasmids were transfected into HEK293T cell type together with pMD2.G (Addgene, #12259) and psPAX2 (Addgene, #12260) by PEI in OptiMEM medium. The virus-containing medium was collected 2 days later and concentrated in PEG-it Virus Precipitation Solution (SBI, LV825A-1) according to the manufacturer's instructions. The concentrated lentiviruses were added into HEK cell culture, facilitated by polybrene (Sigma, H9268). 2 days later, the cells were suspended and selected by 10 µg/mL Blasticidin (ThermoFisher, A1113903).

For AAV packaging, the inventors used HEK293-AAV cell type (Cell Biolabs). The AAV8 serotype helper plasmid was purchased from PlasmidFactory. We transfected AAV-U6-Pi4kb gRNA-TBG-Cre, AAV-U6-Sacm1l gRNA-TBG-Cre, AAV-U6-Cul7 gRNA-TBG-Cre, AAV-U6-Fbxw8 gRNA-TBG-Cre or AAV-U6-NS gRNA-TBG-Cre plasmids into HEK293-AAV cell line via PEI in OptiMEM medium, together with helper plasmid. The medium was collected 4 to 5 days later for concentration via AAVanced Concentration Reagent (SBI, AAV110A-1) according to the manufacturer's instructions. The titer of concentrated viruses was determined by quantitative PCR based on titer determination protocol from Addgene.

### Golgi apparatus and LDs extraction

Minute Golgi Apparatus Enrichment Kit (GO-037, Invent Biotechnologies Inc.) was used to fractionate Golgi apparatus and Golgi related vesicles according to the manufacturer's instructions. Around 3×10⁷ HEK293T cell type was used for fractionation. The Golgi apparatus and vesicle containing pellets were directly dissolved in the RIPA buffer with 10 min sonication before denaturation in Lämmeli buffer. The proteins of interest were analyzed via immunoblotting as described below.

LDs isolation was performed as described previously (Bersuker, K. et al. Developmental Cell 44, (2018)). Briefly, HepG2 cell type was harvested in cold PBS and centrifuged at 500×g for 10 minutes to pellet cells which were incubated in cold hypotonic lysis medium (HLM, 20 mM Tris-HCl pH 7.4 and 1 mM EDTA) protease inhibitors (Complete, Roche). After being homogenized in a dounce, the homogenate was centrifuged at 1000×g for 10 minutes to collect the supernatant for further ultracentrifugation via HLM at different sucrose concentrations. The buoyant fractions were collected and finally suspended in 1% SDS containing RIPA buffer for immunoblotting analyses.

### Immunoblotting, immunoprecipitation, co-immunoprecipitation, and immunofluorescence

Adipose depots, liver pieces, differentiated iBAs, HepG2, HEK293T and HEK293-AAV cell types were homogenized in RIPA buffer (50 mM Tris-HCl pH 7.4, 150 mM NaCl, 2 mM EDTA, 1.0% Triton X100, 0.5% sodium deoxycholate) with protease inhibitors (Complete, Roche) and phosphatase inhibitors (Halt phosphatase inhibitor cocktail, ThermoFisher). The homogenates were centrifuged at 12,000×g for 10 min at 4°C to remove debris and collect whole cell extract (WCE). The concentration of WCE was determined by DC Protein Assay (Bio-Rad). After boiling the sample with Lämmeli buffer at 95°C for 5 min, equal amount of proteins was loaded and separated on 12% SDS-PAGE. The proteins were transferred to nitrocellulose membrane and blotted by ATGL (1:1000, Cell Signaling), CGI-58 (1:1000, Proteintech), phospho-HSL(Ser660) (1:1000, Cell Signaling), PLIN1 (1:1000, Cell Signaling), HSL (1:1000, Cell Signaling), γ-tubulin (1:10000, Sigma), FLAG (1:10000, Sigma; 1:1000, Cell Signaling), MGL (1:1000, Abcam), HA (1:1000, Cell signaling), FBXW8 (1:100, Santa Cruz; 1:1000, Sigma), CUL7 (1:100, Santa Cruz), HSP90 (1:1000, Cell Signaling), K48 polyubiquitin (1:1000, Cell signaling), PI4KB (1:1000, Sigma), SACM1L (1:1000, Sigma), AKT1 (1:1000, Cell Signaling), phospho-AKT1(Ser473) (1:1000, Cell Signaling), TGN46 (1:1000, Abcam), MAOA (1:1000, Abcam), PLIN2 (1:1000, Abcam), PMP70 (1:10000, Sigma), Calnexin (1:1000, Cell Signaling), ARF1 (1:1000, Invitrogen), GAPDH (1:1000, Cell Signaling), RAB7 (1:1000, Cell Signaling), alpha 1 Sodium Potassium ATPase (1:1000, Abcam), Cleaved Caspase-3 (1:1000, Cell Signaling), phospho-AMPKα (Thr172) (1:1000, Cell Signaling), AMPKα (1:1000, Cell Signaling) and GM130 (1:1000, Proteintech). The primay antibody signal was visualized by HRP-conjugated secondary antibodies (1:5000, Millipore) and the ImageQuant system (GE Healthcare Life Sciences).

To immunoprecipitate FLAG tagged ATGL or FBXW8, HEK293 cells were homogenized in RIPA buffer (50 mM Tris-HCl pH 7.4, 150 mM NaCl, 2 mM EDTA, 1.0% Triton X100) with protease inhibitors (Complete, Roche) and phosphatase inhibitors (Halt phosphatase inhibitor cocktail, ThermoFisher). The homogenates were centrifuged at 12,000×g for 10 min at 4°C to remove debris and collect WCE. The concentration of WCE was determined by DC Protein Assay (Bio-Rad). After three washes with cold RIPA buffer, 20 µl Anti-FLAG beads (A2220, Sigma) was added to WCE for incubation with rotating overnight at 4°C. The beads were then washed 6 times by cold RIPA buffer. The proteins were eluted by boiling the beads at 95°C for 5 min with 2×Lämmeli buffer. The eluted protein was then analyzed by immunoblotting through K48 specific ubiquitin antibody (1:1000, Cell Signaling), γ-tubulin (1:10000, Sigma), ATGL (1:1000, Cell Signaling), FBXW8 (1:1000, Sigma) and FLAG (1:1000, Cell Signaling).

To immunostain ATGL, GM130, CUL7, FBXW8 and FBXW8-FLAG in the HEK293-AAV cell type or stable cell line expressing Scarlet-Giantin after various treatments, HEK293-AAV cell type was fixed by 4% PFA for 20 min and permeabilized by 0.25% PBST (Triton X100 in PBS) or 20 µM digitonin (only for ATGL immunostaining) for 20 min. After three washes with cold PBS, cells were blocked by 1% BSA for 1 h and incubated with ATGL antibody (1:100, Cell Signaling), GM130 (1:100, Proteintech), CUL7 antibody (1:50, Santa Cruz), FBXW8 antibody (1:100, Sigma) and FLAG antibody (1:100, Cell Signaling) overnight at 4°C. After three washes with cold PBS, cells were incubated with Alexa Fluor 488 conjugated secondary antibody (1:200, ThermoFisher) for 1 h at room temperature. Finally, cells were stained by Hoechst 33342 to label nuclei after three washes with PBS to remove residual secondary antibody. We determined the ATGL intensity/Golgi area ratio or FBXW8 intensity/Golgi area ratio to quantify the Golgi ATGL or FBXW8 levels respectively and the ratios from the experimental groups were shown as relative value to the control. Likewise, we used the same procedures to immunostain GM130 in the HepG2 cell type except using Alexa Fluor 568 conjugated secondary antibody (1:200, ThermoFisher) for 1 h incubation.

To immunostain ATGL in the iBAT, the tissues were fixed by 4% PFA overnight 4°C after dissection. Then 10% sucrose and 30% sucrose solution were used to dehydrate samples before cryosection at the thickness of 15 µm. The slides were washed once in PBS for 5 min and 3 washes in 0.1% PBST before blocking with 10% normal donkey serum (NDS) diluted in PBST for 1 h. Afterwards, ATGL antibody (1:100, Cell Signaling) was diluted in blocking solution for overnight incubation at 4°C. The residual antibody was washed with PBST three times before application of Alexa Fluor 568 conjugated secondary antibody (1:200 in 5% NDS, ThermoFisher) for 1 h. At last, the slides were treated with mounting solution after three washes with PBST.

The pictures were all obtained by Olympus FluoView 3000 Confocal and processed by Image J.

### Ptdlns4P determination

HEK293-AAV cell type was co-expressed with Scarlet-Giantin and EGFP-P4M, followed by glucose deprivation. The cells were live-imaged and the Golgi Ptdlns4P levels were determined as Golgi GFP intensity/cytosolic GFP intensity ratio in the individual cells. The Golgi Ptdlns4P levels after glucose deprivation were shown as the relative value to the ratio acquired from cells cultured under normal glucose medium.

To immunostain cellular Ptdlns4P at different conditions, HEK293-AAV cell type (Scarlet-Giantin stable cell line) was fixed by 4% PFA for 15 min and washed three times with PBS containing 50 mM NH4Cl, followed by permeabilization for 5 min with 20 µM digitonin in PBS. After being washed three times with PBS, cells were blocked with 5% normal goat serum (NGS) in PBS with 50 mM NH4Cl for 1 h at room temperature. Primary anti-Ptdlns4P antibody (1:100 in 5% NGS; Echelon Biosciences) was applied to cells overnight at 4°C. Alexa Fluor 488 conjugated secondary antibody (1:200 in 5% NGS, ThermoFisher) was replaced after three washes. At last, cells were washed three times and stained by Hoechst 33342 to label nuclei. We determined the Ptdlns4P intensity/Golgi area ratio to quantify the Golgi Ptdlns4P levels and the ratios from the experimental groups were shown as relative value to the control.

Ptdlns4P Mass ELISA Assay Kit (K-4000E, Echelon Biosciences) was used to determine the total mass of Ptdlns4P from HEK293T cell type according to the manufacturer's instructions. Prior to the assay, total acidic lipids were extracted via TCA precipitation, methanol:CHCl₃:HCl (80:40:1) extraction and phase separation. The organic phase was dried, and the remnant was dissolved in PBS-T buffer with 10 min sonication. The lipid samples were then measured and normalized to the total protein mass.

### Membrane Lipid Strip assay, PIP Strip assay and Pull-down by Ptdlns4P agarose beads

FLAG Purification Kit (CELLMM2, Sigma) was used to purify wild type and mutant FBXW8 from established HEK293T stable cell line according to the manufacturer's instructions. After elution with buffer containing 3×FLAG peptide, the elutes were filtered and concentrated using a 10-kDa cut-off concentrator (Pierce, 88513). Before aliquoting the purified recombinant proteins, the concentration of them was determined via Protein Assay (Bio-Rad).

Membrane Lipid Strip (P-6002, Echelon Biosciences) and PIP Strip (P-6001, Echelon Biosciences) were used to perform binding assays between recombinant proteins and lipids. Briefly, both Strip membranes were blocked with Block Buffer (PBS with 0.1% Tween 20 and 3% fat-free BSA) for 1 h. 3 µg recombinant proteins were diluted in fresh Block Buffer and applied to Strip membranes for 1 h. The Strip membranes were then washed three times with Strip Wash Buffer (PBS with 0.1% Tween 20) for 30 min in total. The Strip membranes were incubated with FLAG antibody (1:1000, Cell signaling) diluted in Strip Wash Buffer for 1 h, followed by 3 washes and incubation with HRP-conjugated secondary antibody (1:10000, Cell Signaling) for another 1 hour as regular immunoblotting process. The Strip membranes were developed and captured in the ImageQuant system (GE Healthcare Life Sciences).

Ptdlns4P conjugated beads (P-B004, Echelon Biosciences), PC (Phosphatidylcholine) conjugated beads (P-B0PC, Echelon Biosciences) and control beads (P-B000, Echelon Biosciences) were used to pull down purified recombinant proteins according to manufacturer's instructions. In brief, 100 µl slurry was pelleted by centrifugation at low speed and the pellet was resuspended in 300 µl wash-binding buffer (10 mM HEPES, PH 7.4, 150 mM NaCl, 0.25% Igepal). 10 µg recombinant wild type and mutant FBXW8 were added for 3 h incubation at room temperature. The beads were pelleted and washed with wash-binding buffer 6 times. At last, the proteins were eluted by boiling the beads at 95°C for 5 min with 2×Lämmeli buffer. The eluted proteins were then analyzed by immunoblotting through FLAG antibody (1:1000, Cell Signaling).

### Lipolysis measurement

For *in vitro* lipolysis measurement, day 9 iBAs were washed with pre-warmed PBS and then starved by phenol red free DMEM medium (low glucose) with 1% fatty acid free BSA for 2 h. After medium collection, non-esterified fatty acid release was measured with NEFA assay kit (Wako NEFA kit) via Synergy Gen5 Platereader and normalized to total protein mass. Similarly, the dialysate from liver graft *ex vivo* perfusion collected at different timepoints was measured with the same kit to determine NEFA levels.

### Plasma insulin and glucose measurement

For mouse insulin measurement, plasma was collected at different timepoints after 2-DG administration in wild type mice. 5 µl plasma sample was used to determine insulin levels via Ultra-sensitive mouse insulin ELISA kit (Crystal Chem, Cat# 90080) according to the manufacturer's protocol. The absorbance was measured via Synergy Gen5 Platereader at 450/630 nm. 2 µl plasma sample was used to determine glucose levels via Glucose Assay Kit (Sigma, MAK263) according to the manufacturer's protocol. The absorbance was read at 570 nm in Synergy Gen5 Platereader.

### Levels of FFA, TG, ALT and AST activity in the plasma

Plasma collected from mice under different conditions was used to determine levels of lipolysis and liver injury. FFA levels were measured via NEFA assay kit (Wako NEFA kit). TG levels were determined via TG assay kit (Roche Trig/GB reagent). ALT (MAK052, Sigma) and AST (MAK055, Sigma) assay kits were used to determine ALT and AST activity according to the manufacturer's instructions. The absorbance was monitored using Synergy Gen5 Platereader.

### Glucose, glucose-6-phosphate and glyceraldehyde 3-phosphate determination in the liver

Liver pieces (50-100 mg) were homogenized in 2 volumes of ice-cold PBS. The homogenized samples were centrifuged at 13000×g for 10 minutes to remove insoluble material. The supernatant was collected, and the protein concentration was measured via Protein Assay (Bio-Rad). Afterwards, the supernatant was deproteinized with a 10 kDa MWCO spin filter (Pierce, #88513). 1 µl deproteinized sample was used to measure glucose levels via Glucose Assay Kit (Sigma, MAK263). 10 ul sample was used to measure glucose-6-phosphate levels via Glucose-6-Phosphate Assay Kit (Sigma, MAK014) at 450 nm via Synergy Gen5 Platereader, or to measure glyceraldehyde 3-phosphate levels via Glyceraldehyde 3-Phosphate Assay Kit (Abcam, ab273344) via recording fluorescence in the kinetic mode every 30 seconds at 535/587 nm in Synergy Gen5 Platereader. The concentration of glucose, glucose-6-phosphate and glyceraldehyde 3-phosphate were normalized to the protein concentration.

### Liver TG determination

Weight of liver pieces (50-100 mg) was recorded before homogenization by 1 mL isopropanol per 50 mg tissue. The homogenates were incubated at room temperature with rotating before spinning down to remove debris at 2000×g for 10 min and collect supernatant. The TG levels were determined (Roche Trig/GB reagent) in Synergy Gen5 Platereader and normalized to the weight of liver pieces.

### Histologic staining

Livers from mice under normal chow diet and NAFLD mice were fixed in 4% PFA in PBS for 24 h directly after tissue harvesting. To perform hematoxylin and eosin (H&E) staining, the fixed liver pieces were transferred to 65% ethanol and embedded in paraffin in tissue processing machine and embedding machine, followed by section at 10 µm and staining using the standard protocol. To perform Oil Red O staining, the fixed livers were sequentially dehydrated with 10% sucrose and 30% sucrose solution. The liver pieces underwent cryosection at 20 µm and staining via Oil Red O solution (01516, Sigma).

For NASH cohort, livers were dissected and fixed with 4% PFA in PBS for 24 h at 4°C. The samples were sent to the University of Zurich for histologic analyses. Briefly, liver tissues were dehydrated and embedded in paraffin and underwent section at 3 µm. The slides were stained with hematoxylin and eosin (H&E) and Gomori Green Trichrome directly or stained with anti-cleaved Caspase 3 after antigen retrieval. Afterwards, the images were captured by digital slide scanners and the grade of steatosis, ballooning, inflammation and fibrosis were quantified via the NASH Clinical Research Network Scoring System (Kleiner,D.E. et al. Hepatology 41, (2005)). To perform ORO staining, the fixed samples were sectioned, and the slides were stained via Oil Red O solution, followed by counter-staining with eosin. The images were acquired by digital slide scanners.

### Quantification and statistical analysis

For *in vivo* studies, littermates were randomly assigned to treatment groups for all the experiments. Sample size was determined based on previous experiments and the animal numbers used in the experiments were all indicated in the corresponding figure legends. For cell culture experiments, biological replicates were no less than 3 (shown as n in the figure legend) and all the cell culture experiments were performed with 2-3 technical replicates for RNA and protein analysis. Results are represented as mean ± s.e.m. when it is not indicated specifically. Two-tailed unpaired or paired Student's *t*-test was applied on comparison of two groups when the data are in normal distribution, otherwise Mann-Whitney test or Wilcoxon test was adopted. ANOVA was applied on comparisons of multiple groups when the data are in normal distribution, otherwise Kruskal-Wallis test was adopted. All statistical analyses were performed using GraphPad Prism 7 software. Statistical differences are indicated as exact *p* value or nonsignificant (n.s.).

### Cited references:

Ding, L. et al. Peroxisomal β-oxidation acts as a sensor for intracellular fatty acids and regulates lipolysis. Nat Metab 1-14 (2021) doi:10.1038/s42255-021-00489-2.
Loomba, R., Friedman, S. L. & Shulman, G. I. Mechanisms and disease consequences of nonalcoholic fatty liver disease. Cell 184, 2537-2564 (2021).
Clavien, P.-A. et al. Transplantation of a human liver following 3 days of ex situ normothermic preservation. Nat Biotechnol 40, 1610-1616 (2022).
Eshmuminov, D. et al. An integrated perfusion machine preserves injured human livers for 1 week. Nat Biotechnol 38, 189-198 (2020).
Sitnick, M. T. et al. Skeletal Muscle Triacylglycerol Hydrolysis Does Not Influence Metabolic Complications of Obesity. Diabetes 62, 3350-3361 (2013).
Klein, J., Fasshauer, M., Klein, H. H., Benito, M. & Kahn, C. R. Novel adipocyte lines from brown fat: A model system for the study of differentiation, energy metabolism, and insulin action. BioEssays 24, 382-388 (2002).
Renella et al. A multisociety Delphi consensus statement on new fatty liver disease nomenclature. Hepatology 78(6), 1966-1986 (2023).

All scientific publications and patent documents cited in the present specification are incorporated by reference herein. Particularly, EP 2 935 269 B1, EP 2 193 131 B1 and Knight et al. 1006, Cell 125:733-747 are incorporate by reference herein.

### SEQUENCES:

In the event of discrepancies between the sequences shown in the present specification and those of the enclosed sequence protocol according to WIPO standard ST.26, the sequences shown herein shall prevail.

## Claims

1. A method for defattening a steatotic liver *ex vivo,* the method comprising
- perfusing a steatotic liver graft in a perfusion step using a perfusate comprising a compound selected from a PI4KB inhibitor of formula 1, 2, and 3, or a pharmaceutically acceptable salt or solvate thereof, 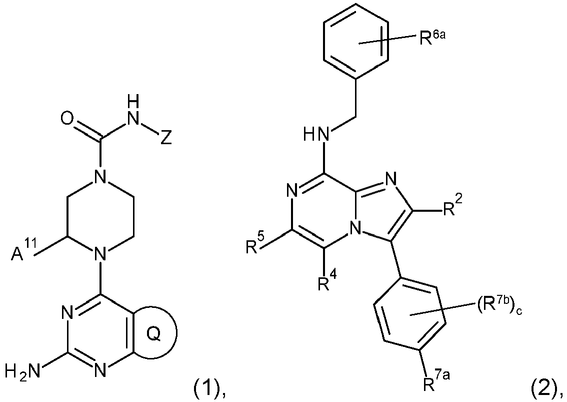 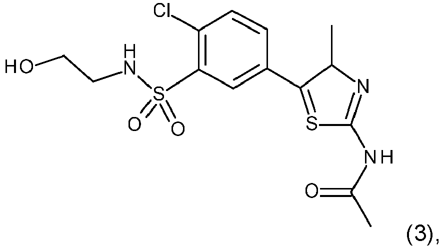 wherein
- Z is (methoxy)(methyl)phenyl, (methyl)(trifluoromethoxy)phenyl, (methoxy)(methyl)pyridinyl, (ethyl)(methoxy)pyridinyl, (ethoxy)(methyl)pyridinyl or dimethoxypyridinyl,
- A¹¹ is H, methyl, ethyl, hydroxymethyl or hydroxyethyl,
- Q is a heterocycle of formula 4, 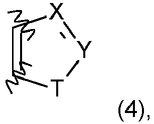 wherein
Xis N or C(H),
Y is N or C(H),
T is S or N(CH₃)
- R², R⁴ and R⁵ are independently selected from H, methyl and ethyl,
- R^{6a} is SO₂R⁸, wherein R^{6a} is in para or meta position,
- R^{7a} is selected from OH, OCH₃, halogen, NH₂, CH₂-OH and NH-CO-R⁸,
- each R^{7b} is independently selected from OH, halogen, CN, NO₂, C₁-C₆ alkyl, C₁-C₆ alkoxy, OCF₃, OCH₂CF₃, OCH₂Ph, OCH₂-cyclopropyl, OCH₂CH₂OH, OCH₂CH₂NMe₂, CF₃, OCF₃, C₁-C₆ alkyl-OH, NHSO₂R⁹, NHCOR⁸ and NR⁹R¹⁰,
- c is 0 or 1
wherein
R⁸ is C₁-C₆ alkyl or NR⁹R¹⁰, and
R⁹ and R¹⁰ are independently selected from H and C₁-₆ alkyl.

2. The method according to claim 1, wherein the concentration of the PI4KB inhibitor in the perfusate is 0.1 to 100 µM.

3. The method according to claim 3, wherein the initial concentration of the PI4KB inhibitor in the perfusate is 0.1 to 100 µM.

4. The method according to claim 2, wherein the concentration of the PI4KB inhibitor is controlled and adjusted during the perfusion step to a concentration in the perfusate of 1 to 40 µM.

5. The method according to any of the preceding, wherein the concentration of the PI4KB inhibitor is adjusted by
- adding a bolus of the PI4KB inhibitor, wherein the PI4KB inhibitor is dissolved in an organic solvent, particularly DMSO, or
- continuous administration, wherein the PI4KB inhibitor is bound to albumin, or the PI4KB inhibitor is encapsulated in nanoemulsion, or the PI4KB inhibitor is part of a nanoparticle.

6. The method according to any of the preceding claims, wherein the perfusion step is performed until a non-steatotic liver is obtained, particularly the perfusion step is performed for 2 to 14 days, more particularly for 8 days.

7. The method according to any of the preceding claims, wherein the perfusate comprises a perfusion medium, wherein the perfusion medium is whole blood, leukocyte-depleted blood, leukocyte-free blood, or an oxygen-carrying blood substitute.

8. The method according to claim 1, wherein the compound is selected from a PI4KB inhibitor of formula 1a, 2a, and 3, 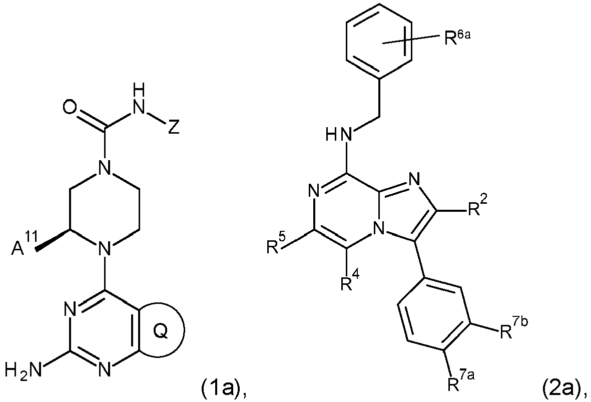 wherein Z, A¹¹, Q, R², R4, R⁵, R^{6a}, R^{7a}, R^{7b} and c are defined as described above.

9. The compound for use according to any of the preceding claims, wherein A¹¹ is methyl, ethyl, hydroxymethyl or hydroxyethyl.

10. The compound for use according to any of the preceding claims, wherein Z is selected from a moiety of formula Za, Zb, Zc, Zd, Ze, Zf, Zg, Zh and Zi,

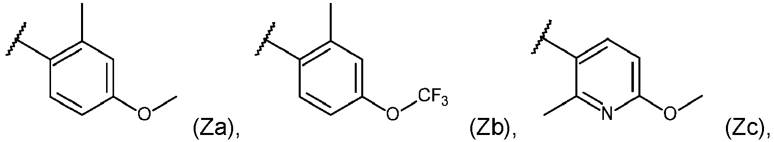

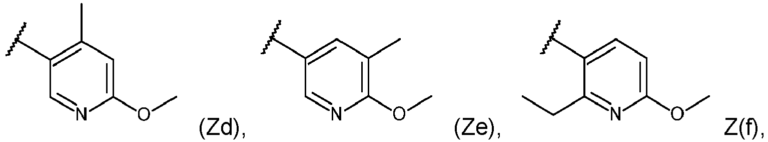

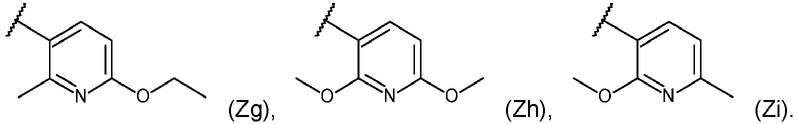

11. The compound for use according to any of the preceding claims, wherein one of X and Y is N and the other is C(H).

12. The compound for use according to any of the preceding claims, wherein Q is selected from a heterocycle of formula Qa, Qb, Qc, Qd and Qe,

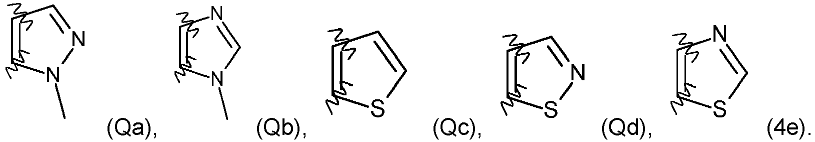

13. The compound for use according to any of the preceding claims, wherein R^{6a} is So₂R⁸, wherein R⁸ is a C₁-C₆ alkyl.

14. The compound for use according to any of the preceding claims, wherein
- R^{7a} is selected from OH, NH₂ and OCH₃, and/or
- R^{7b} is selected from OH, halogen, NO₂, C₁-C₆ alkyl, C₁-C₆ alkoxy, C₁-C₆ alkyl-OH, NH₂, NHSO₂R⁹ and NHCOR⁸ with R⁹ and R⁸ being defined as described above.

15. The compound for use according to any of the preceding claims, wherein the compound is selected from a PI4KB inhibitor of formula 1b, 2b and 3,
